(19) Europäisches Patentamt
European Patent Office
Office européen des brevets

(11) **EP 3 711 660 B1**

(12) **EUROPÄISCHE PATENTSCHRIFT**

(45) Veröffentlichungstag und Bekanntmachung des Hinweises auf die Patenterteilung:
**03.11.2021  Patentblatt 2021/44**

(21) Anmeldenummer: **20173941.4**

(22) Anmeldetag: **16.08.2017**

(51) Int Cl.:
*A61B 5/024* (2006.01)     *A47G 19/00* (2006.01)
*A47G 23/00* (2006.01)     *A61B 5/00* (2006.01)
*G01F 23/00* (2006.01)     *A61B 5/021* (2006.01)
*A47G 19/22* (2006.01)     *A47G 23/16* (2006.01)

(54) **SYSTEM ZUM ÜBERWACHEN EINER FLÜSSIGKEITSAUFNAHME EINES BENUTZERS**

SYSTEM FOR MONITORING A FLUID INTAKE OF A USER

SYSTÈME DE SURVEILLANCE D'UN APPORT EN LIQUIDE D'UN UTILISATEUR

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **19.08.2016  DE 102016215615**

(43) Veröffentlichungstag der Anmeldung:
**23.09.2020  Patentblatt 2020/39**

(62) Dokumentnummer(n) der früheren Anmeldung(en) nach Art. 76 EPÜ:
**17761029.2 / 3 500 164**

(73) Patentinhaber: **Belenus Verwaltungsgesellschaft mbH**
**81243 München (DE)**

(72) Erfinder:
• **Hoffmann, Bernd**
**81243 München (DE)**

• **Heinicke, Susann**
**81243 München (DE)**

(74) Vertreter: **Schenk, Markus**
**Schoppe, Zimmermann, Stöckeler Zinkler, Schenk & Partner mbB Patentanwälte**
**Radlkoferstrasse 2**
**81373 München (DE)**

(56) Entgegenhaltungen:
**US-A1- 2012 094 261     US-A1- 2014 046 596**
**US-A1- 2015 359 364     US-A1- 2016 143 583**

• **Henry Hopper: "A Dozen Ways to Measure Fluid Level and How They Work | Sensors Magazine", , 1. Dezember 2004 (2004-12-01), XP055443764, Gefunden im Internet: URL:https://www.sensorsmag.com/components / a-dozen-ways-to-measure-fluid-level-and-ho w-they-work [gefunden am 2018-01-23]**

**Beschreibung**

Die vorliegende Erfindung bezieht sich auf ein System zum Überwachen einer Flüssigkeitsaufnahme eines Benutzers. Ausführungsbeispiele zeigen einen Behälter zum Aufnehmen von Flüssigkeit mit einem Füllstandsmesser zum Messen eines Füllstands der in dem Behälter befindlichen Flüssigkeit.

Bisherige Systeme zur Überwachung der Flüssigkeitsaufnahme ermöglichen eine allgemeine bzw. durchschnittliche Bestimmung der Flüssigkeitsaufnahme ausschließlich basierend auf einer dem Behälter zugeführten oder entnommenen Flüssigkeitsmenge. Hierfür kann der Behälter beispielsweise auf einem Untersetzer stehen, in dem die Auswertung durchgeführt wird. Hiermit kann jedoch keine personenspezifische Flüssigkeitsaufnahme bestimmt werden. Ebenso ist das System sehr fehleranfällig, da das Glas versehentlich oder beispielsweise aufgrund von Vergesslichkeit z. B. bei älteren Menschen neben den Untersetzer gestellt wird und somit eine genaue Füllstandsinformation des Glases nicht durchgängig bestimmt werden kann. US 2014/046596 A1 und US 2015/359364 A1 sind Beispiele von Systemen mit Füllstandmessern zum Animieren zum Trinken.

Der vorliegenden Erfindung liegt somit die Aufgabe zugrunde, ein verbessertes Konzept zur Überwachung einer aufgenommenen Flüssigkeitsmenge eines Benutzers zu schaffen. Diese Aufgabe wird durch den Gegenstand der unabhängigen Patentansprüche gelöst. Erfindungsgemäße Weiterbildungen sind in den Unteransprüchen definiert.

Ausführungsbeispiele eines ersten Aspekts betreffen ein System zum Überwachen einer Flüssigkeitsaufnahme eines Benutzers mit einem Behälter zum Aufnehmen von Flüssigkeit, mit einem Füllstandsmesser zum Messen des Füllstands der in dem Behälter befindlichen Flüssigkeit, mit einer Überwachungseinrichtung, die ausgebildet ist, um eine aus dem Behälter entnommene Flüssigkeitsmenge zu bestimmen, und mit einer Animationseinrichtung, die ausgebildet ist, um abhängig von der entnommenen Flüssigkeitsmenge den Benutzer zum Trinken zu animieren.

Insbesondere betrifft die vorliegende Erfindung ein System zum Überwachen einer Flüssigkeitsaufnahme eines Benutzers mit einem Behälter zum Aufnehmen von Flüssigkeit und mit einem Füllstandsmesser zum Messen des Füllstands der in dem Behälter befindlichen Flüssigkeit, wobei der Füllstandsmesser ein optischer Füllstandsmesser ist, der mindestens einen Emitter zum Emittieren von elektromagnetischer Strahlung und mindestens einen Empfänger zum Empfangen der emittierten elektromagnetischen Strahlung aufweist. Das System weist ferner eine Überwachungseinrichtung auf, die ausgebildet ist, um eine aus dem Behälter entnommene Flüssigkeitsmenge zu bestimmen, sowie eine Animationseinrichtung, die ausgebildet ist, um abhängig von der entnommenen Flüssigkeitsmenge den Benutzer zum Trinken zu animieren. Erfindungsgemäß ist der Emitter derart an dem Behälter angeordnet, dass die emittierte elektromagnetische Strahlung auf die zur Umgebung angrenzende Flüssigkeitsoberfläche der in dem Behälter befindlichen Flüssigkeit auftrifft, wobei der Empfänger derart an dem Behälter angeordnet ist, dass ein an der Flüssigkeitsoberfläche reflektierter Anteil der vom Emitter emittierten elektromagnetischen Strahlung von dem Empfänger empfangbar ist, und wobei zumindest einer von dem Emitter und dem Empfänger an einem Behälterboden des Behälters angeordnet ist.

Ausführungsbeispiele eines zweiten Aspekts betreffen ein System zum Überwachen einer Flüssigkeitsaufnahme eines Benutzers mit einer Überwachungseinrichtung, einer Schnittstelle und einer Animationseinrichtung. Die Überwachungseinrichtung kann eine aus einem Behälter entnommene Flüssigkeitsmenge bestimmen. Die Schnittstelle ist ausgebildet, um von einem Vitalparametersensor einen Vitalparameter eines Benutzers des Systems zu empfangen. Ferner kann die Animationseinrichtung abhängig von der entnommenen Flüssigkeitsmenge und dem Vitalparameter oder alternativ einer Mehrzahl von Vitalparametern den Benutzer zum Trinken animieren.

Diesem zweiten Aspekt liegt die Idee zugrunde, eine personenspezifische bzw. individuelle optimale Flüssigkeitsmenge für einen Benutzer unter Einbeziehung von einer (kontinuierlichen) Messung von Vitalparametern des Benutzers zu bestimmen. Ferner ist das System in der Lage, sich adaptiv an die aktuellen Lebensumstände der Person anzupassen. So kann z. B. über die Pulsfrequenz oder den abgesonderten Schweiß festgestellt werden, ob eine Person Sport treibt und/oder viel schwitzt und somit einen erhöhten Flüssigkeitsbedarf hat oder ob die Person z. B. schläft und somit einen geringeren Flüssigkeitsbedarf aufweist. Dies ist möglich, indem die Berechnung der aufgenommenen Flüssigkeitsmenge unter Berücksichtigung von bereitgestellten Vitalparametern aufgezeichnet wird, um so, basierend auf einem ermittelten Flüssigkeitsbedarf und der tatsächlich aufgenommenen Flüssigkeitsmenge die aufzunehmende Flüssigkeitsmenge zu berechnen. Überschreitet die bestimmte, aufzunehmende Flüssigkeitsmenge einen Grenzwert bzw. einen Schwellenwert, so wird der Benutzer zum Trinken animiert.

Ausführungsbeispiele zeigen, dass die Überwachungseinrichtung, die Schnittstelle und die Animationseinrichtung in einem Sockel angeordnet sind, der ausgebildet ist, um den Behälter aufzunehmen. Somit kann der Sockel die Berechnung übernehmen, ob der Schwellenwert zur Signalisierung, dass Flüssigkeit durch den Benutzer aufgenommen werden soll, bzw. zur Trinkanimation sowie die Animation des Benutzers an sich ausführen.

Ausführungsbeispiele eines dritten Aspekts betreffen ein System zum Überwachen einer Flüssigkeitsaufnahme eines Benutzers mit einem Behälter, einem Sockel, einer Überwachungseinrichtung und einer Animationseinrichtung. Der Behälter weist an einer Auflagefläche einen Vorsprung oder eine Wölbung auf, so dass eine Stabilität des Behälters beim Abstellen des Behälters auf die Auflagefläche reduziert ist. Der Sockel ist ausgebildet, um den Behälter aufzunehmen, so dass der Behälter in Verbindung mit dem Sockel stabil abstellbar ist. Die Überwachungseinrichtung bestimmt die aus dem Behälter entnommene Flüssigkeitsmenge, wobei die Animationseinrichtung ausgebildet ist, um abhängig von der entnommenen Flüssigkeitsmenge, den Benutzer zum Trinken zu animieren.

Dies ist vorteilhaft, da ein versehentliches Abstellen oder ein Benutzen des Behälters ohne den Sockel, in dem zumindest die Erfassung einer aktuellen Flüssigkeitsmenge in dem Behälter stattfindet, vermieden werden kann. Somit ist sichergestellt, dass der Behälter ausschließlich in Verbindung mit dem Sockel benutzt wird und Lücken bei der Überwachung der Flüssigkeitsaufnahme durch die Person verhindert werden bzw. nicht entstehen. In den Ausführungsbeispielen weist das System gemäß dem dritten Aspekt vorteilhafterweise eine Schnittstelle auf, die an einem Vitalparametersensor einen Vitalparameter eines Benutzers des Systems empfangen kann, wobei die Animationseinrichtung ferner ausgebildet ist, um abhängig von der entnommenen Flüssigkeitsmenge und dem Vitalparameter, den Benutzer zum Trinken zu animieren. Die Überwachungseinrichtung und die Animationseinrichtung kann gemäß einem Ausführungsbeispiel in dem Sockel ausgebildet sein. Dies ist vorteilhaft, da somit gemäß dem zweiten Aspekt eine adaptive und personenspezifische Bestimmung der benötigten Flüssigkeitsmenge durchgeführt werden kann, und die bereits aufgenommene Flüssigkeitsmenge nicht durch eine Fehlbedienung bzw. -behandlung des Systems verfälscht werden kann, da der Becher ohne den Sockel nicht oder nur schwer abstellbar ist.

So kann eine effektive Aufstellfläche des Behälters beispielsweise um mehr als 60%, mehr als 75% oder mehr als 90% gegenüber einer maximalen Querschnittsfläche eines Bereichs des Behälters, der eine Flüssigkeit aufnehmen kann, reduziert werden. Alternativ zu der maximalen Fläche kann auch eine durchschnittliche Querschnittsfläche des Flüssigkeitsaufnahmebereichs des Behälters als Vergleichswert herangezogen werden, auf den sich die Reduzierung der effektiven Aufstandsfläche bezieht.

Die weiteren Ausführungsbeispiele stellen vorteilhafte Ausgestaltungen hinsichtlich der drei vorgenannten Aspekte dar. So zeigen Ausführungsbeispiele, dass sie die Animationseinrichtung, den Behälter, den Vitalparametersensor oder einen Transceiver bzw. ein mobiles Gerät animieren kann, um den Benutzer visuell, auditiv oder taktil zum Trinken zu animieren. Hinsichtlich einer visuellen Animation kann der Behälter beispielsweise aus einem lichtdurchlässigen Material gebildet sein, wobei die Auswerteeinheit ausgebildet ist, zum Animieren (des Behälters) Licht in den Behälter einzukoppeln. Ferner kann der Behälter Streuzentren aufweisen, die ausgebildet sind, um das eingekoppelte Licht zu streuen und somit die visuelle Animation zu verstärken. Gemäß weiteren Ausführungsbeispielen kann der Behälter auch einen Vorsprung aufweisen, in den die Auswerteeinheit das Licht in den Behälter einkoppelt. Dies ist vorteilhaft, da somit die Einkopplung des Lichts senkrecht zu einer Blickrichtung des Benutzers, der in den Behälter hineinblickt, bzw. senkrecht zu einer Öffnungsrichtung des Behälters erfolgt, und der Nutzer somit nicht von der einkoppelnden Lichtquelle gestört bzw. geblendet wird. Da beim Trinken der Blick des Nutzers nahezu senkrecht ins Glas fällt, kann bei einer Anordnung der LED parallel zu der Öffnungsrichtung des Behälters zum Blenden des Nutzers führen. Um dies zu vermeiden, kann die LED wie beschrieben auch horizontal bzw. senkrecht oder diagonal angebracht werden.

Gemäß weiteren Ausführungsbeispielen kann der Sockel einen Befestigungsmechanismus aufweisen, der den Behälter in einem Betriebszustand z. B. mechanisch oder magnetisch fest mit dem Sockel verbindet. Dies ist vorteilhaft, da somit eine Fehlbenutzung des Systems vermieden wird, indem der Benutzer Flüssigkeit aus dem Behälter entnimmt bzw. trinkt, diese Entnahme jedoch von der Überwachungseinrichtung bzw. Sensoren nicht erfasst wird. Der Behälter ist dann nicht durchgehend mit der Auswerteeinheit verbunden und erschwert oder verhindert somit eine kontinuierliche Füllstandsmessung des Behälters. Gemäß weiteren Ausführungsbeispielen kann der Sockel eine Detektionseinheit aufweisen, die ausgebildet ist, um den Behälter in einem Betriebszustand zu identifizieren und gegenüber weiteren Behältern mit jeweils einer spezifischen Füllmenge zu unterscheiden. Die Unterscheidung der Überwachungseinrichtung ermöglicht, eine behälterspezifische entnommene Flüssigkeitsmenge zu bestimmen und den Behälter dem zugehörigen Benutzer zuzuordnen. Dies ist vorteilhaft, da somit die entnommene Flüssigkeitsmenge nicht sockelspezifisch gespeichert, sondern in Verbindung mit (bzw. in) beispielsweise einem Benutzerprofil gespeichert wird, so dass ein Identifikationsmerkmal, z. B. ein elektronischer Chip, von dem Sockel identifiziert und bei einem (versehentlichen) Tausch der Sockel von zwei oder mehreren Behältern, die korrekte getrunkene Flüssigkeitsmenge dem zu dem Behälter zugehörigen Benutzer zugeordnet wird und nicht dem Nutzer, der den Sockel vorher benutzt hat.

Gemäß weiteren Ausführungsbeispielen weist das System einen Neigungssensor bzw. Neigungsmesser auf, der ausgebildet ist, um einen Neigungswinkel des Behälters zu erfassen. Dies ist vorteilhaft, z. B. wenn die entnommene Flüssigkeitsmenge basierend auf einem Gewicht der Flüssigkeit bestimmt wird. Durch das Anheben und Neigen des Behälters (mit oder ohne den Sockel) kann für diesen Zeitraum keine genaue Bestimmung der im Behälter vorhandenen Flüssigkeitsmenge erfolgen. Somit wird dieser (zeitliche) Bereich von der Überwachung der Füllstandsmenge ausgenommen. Ein Vergleich zwischen der Flüssigkeitsmenge vor dem Anheben bzw. Neigen und ein Vergleich nach dem Abstellen kann die zwischenzeitlich entnommene oder zugeführte Flüssigkeitsmenge wieder konsolidieren bzw. bestimmen. Das detektieren eines Kippen des Behälters kann auch ein Schutz vor Fehlmessungen sein, wenn Flüssigkeit bewusst oder unbewusst (beispielsweise wenn der Behälter umgestoßen wird) verschüttet bzw. ausgegossen wird. Unterstützend bei der Verifizierung kann hier ggf. ein kombinierter Chip zur Neigungs-/Beschleunigungsmessung sein.

Dies ist vorteilhaft, da so die Messung der aktuellen Flüssigkeitsaufnahme nochmals verbessert wird.

Bevorzugte Ausführungsbeispiele der vorliegenden Anmeldung werden nachfolgend Bezug nehmend auf die beiliegenden Zeichnungen näher erläutert. Es zeigen:

Fig. 1       eine schematische Darstellung des Systems zur Überwachung der Aufnahme einer Flüssigkeitsmenge gemäß einem ersten Aspekt,

Fig. 2       eine schematische Darstellung eines Systems zur Überwachung einer entnommenen Flüssigkeitsmenge durch einen Benutzer gemäß einem zweiten Aspekt,

Figs. 3A-3C       eine schematische Darstellung von möglichen Formen eines Behälters mit reduzierter Standfläche,

Fig. 4       eine schematische Darstellung des Systems gemäß einem Ausführungsbeispiel,

Fig. 5       eine schematische Darstellung des Systems mit einem mobilen Gerät,

Figs. 6A-6E       eine schematische Darstellung verschiedener Sensoranordnungen zur Bestimmung der Flüssigkeitsmenge in dem Behälter,

Fig. 7       eine schematische Darstellung des Systems zur Einkopplung von Licht in einen Vorsprung des Behälters,

Fig. 8       eine schematische Querschnittsansicht von einem weiteren Ausführungsbeispiel des Systems, wobei unabhängig von dem gewählten Ausführungsbeispiel eine Adaption des Sockels auf verschiedene Behältergrößen durch das Anbringen eines Erweiterungselements vorgenommen werden kann,

Fig. 9       einen Behälter einer zweiten Größe im Vergleich zu Figur 8,

Fig. 10       eine schematische Darstellung des Systems gemäß einem Ausführungsbeispiel,

Fig. 11       eine Querschnittsansicht eines Behälters für ein System gemäß einem Ausführungsbeispiel,

Fig. 12A       eine Draufsicht auf einen Behälter für ein System gemäß einem Ausführungsbeispiel,

Fig. 12B       eine Draufsicht auf einen Behälter für ein System gemäß einem weiteren Ausführungsbeispiel,

Fig. 13A       eine Querschnittsansicht eines Behälters für ein System gemäß einem weiteren Ausführungsbeispiel,

Fig. 13B       eine schematische Draufsicht auf einen Emitter und einen Empfänger eines Füllstandsmessers für ein System gemäß eines Ausführungsbeispiels,

Fig. 14A       einen Behälter mit Füllstandsmesser gemäß eines Ausführungsbeispiels,

Fig. 14B       den Behälter aus Figur 14A mit einem geringeren Füllstand der in dem Behälter befindlichen Flüssigkeit,

Fig. 15A       eine Querschnittsansicht eines Behälters mit einem Flüssigkeitsdetektor für ein System gemäß eines Ausführungsbeispiels,

Fig. 15B       eine Querschnittsansicht eines Behälters mit einem Flüssigkeitsdetektor für ein System gemäß eines weiteren Ausführungsbeispiels,

Fig. 15C       eine Querschnittsansicht eines Behälters mit einem Flüssigkeitsdetektor für ein System gemäß eines weiteren Ausführungsbeispiels,

Fig. 16       ein Flussdiagramm eines Verfahrens zum Betrieb eines Systems zum Überwachen einer Flüssigkeitsaufnahme eines Benutzers gemäß einem ersten Aspekt, und

Fig. 17       ein Flussdiagramm eines Verfahrens zum Betrieb eines Systems zur Überwachung einer Flüssigkeitsaufnahme eines Benutzers gemäß einem zweiten Aspekt.

In der nachfolgenden Beschreibung der Figuren werden gleiche oder gleichwirkende Elemente mit den gleichen Bezugszeichen versehen, so dass deren Beschreibung in den unterschiedlichen Ausführungsbeispielen untereinander austauschbar ist.

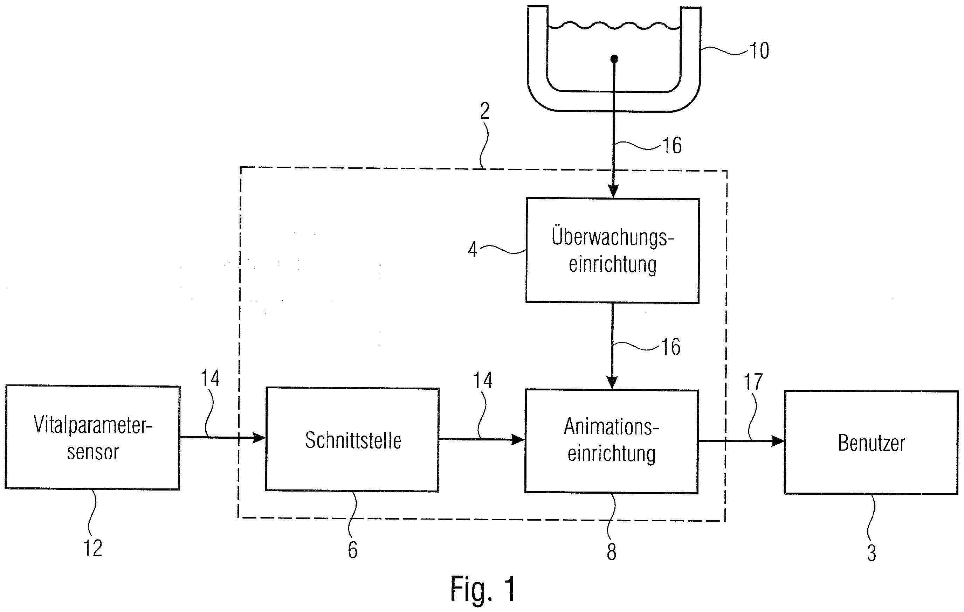

Fig. 1 zeigt eine schematische Darstellung eines Systems 2 zum Überwachen einer Flüssigkeitsaufnahme eines Benutzers 3. Das System 2 weist eine Überwachungseinrichtung 4, eine Schnittstelle 6 und eine Animationseinrichtung 8 auf.

Die Überwachungseinrichtung 4 ist ausgebildet, um eine aus einem Behälter 10 entnommene Flüssigkeitsmenge 16 zu bestimmen. Die Schnittstelle 6, beispielsweise eine Drahtlosschnittstelle, ist ausgebildet, um von einem Vitalparametersensor 12 einen Vitalparameter 14 des Benutzers 3 des Systems 2 zu empfangen.

Ferner ist die Animationseinrichtung 8 ausgebildet, um abhängig von der entnommenen Flüssigkeitsmenge 16 und dem Vitalparameter 14, den Benutzer 3 zum Trinken zu animieren.

Der Vitalparametersensor 12 ist beispielsweise ein Pulsmesser, ein Blutdruckmesser, ein Schweißdetektor, ein Schrittzähler usw.

Ferner kann der Behälter 10 gemäß einem bevorzugten Ausführungsbeispiel in einem passenden Sockel abgestellt werden, wobei der Sockel die Überwachungseinrichtung 4, die Schnittstelle 6 und/oder die Animationseinrichtung 8 aufweisen kann.

Der Schweiß- bzw. Schwitzsensor misst z. B. eine Dehydrierung des Körpers, um basierend auf der Menge des abgegebenen Schweißes und/oder einer Analyse (der Zusammensetzung) des Schweißes eine sehr exakte Bestimmung der vom Körper benötigten Flüssigkeitsmenge zu bestimmen. Implizit kann diese Information beispielsweise auch aus der Bewegung bzw. einem Bewegungsprofil und dem daraus abgeleiteten Kalorienverbrauch berechnet werden.

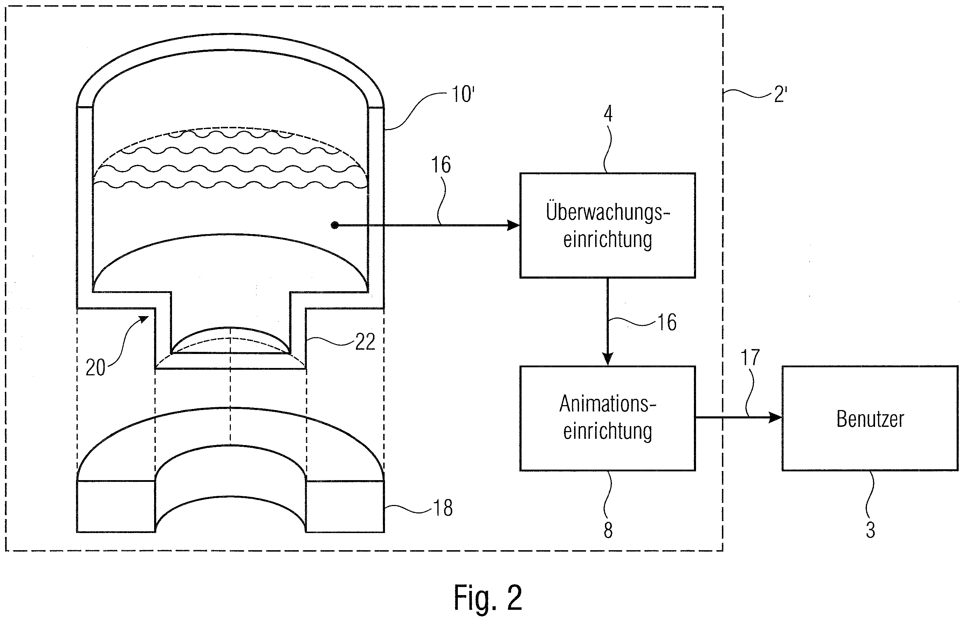

Fig. 2 zeigt eine schematische Darstellung eines Systems 2' zum Überwachen einer Flüssigkeitsaufnahme eines Benutzers 3. Das System 2' weist einen Behälter 10', einen Sockel 18 sowie die Überwachungseinrichtung 4 und die Animationseinrichtung 8 auf.

Der Behälter 10' weist an einer Auflagefläche 20 einen Vorsprung 22 oder eine Wölbung 22 auf, so dass eine Stabilität des Behälters 10' beim Abstellen des Behälters 10' auf die Auflagefläche 20 verringert ist. Ferner ist der Sockel 18 ausgebildet, um den Behälter 10' aufzunehmen, so dass der Behälter 10' in Verbindung mit dem Sockel 18 stabil abstellbar ist.

Die Überwachungseinrichtung 4 ist ausgebildet, um eine aus dem Behälter 10' entnommene Flüssigkeitsmenge zu bestimmen und die Animationseinrichtung 8 ist ferner ausgebildet, um abhängig von der entnommenen Flüssigkeitsmenge 16 den Benutzer 3 zum Trinken zu animieren 17.

Gemäß Ausführungsbeispielen kann das System 2' ferner eine Schnittstelle aufweisen, die ausgebildet ist, um von einem Vitalparametersensor 12 einen Vitalparameter eines Benutzersystems zu empfangen, wobei die Animationseinrichtung 8 ausgebildet ist, um abhängig von der entnommenen Flüssigkeitsmenge und dem Vitalparameter, den Benutzer zum Trinken zu animieren.

Ferner können die Überwachungseinrichtung 4 und die Animationseinrichtung 8 in dem Sockel 18 ausgebildet sein. Somit können in diesem Ausführungsbeispiel die Merkmale und somit auch die Vorteile des System 2 und des Systems 2' vereint werden.

Die nachfolgenden Ausführungsbeispiele beziehen sich sowohl auf das System 2 gemäß dem ersten Aspekt aus Fig. 1 als auch auf das System 2' gemäß dem zweiten Aspekt aus Fig. 2. Ferner sind die Bezugszeichen für das System 2 bzw. 2' und den Behälter 10 bzw. 10' im Folgenden untereinander austauschbar, sofern nicht explizit etwas anderes beschrieben ist.

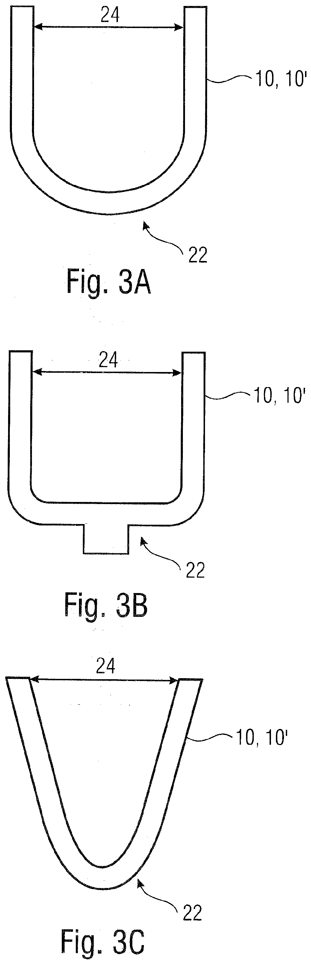

So zeigt beispielsweise Fig. 3 unterschiedliche Formen des Behälters 10, 10', wie er mit dem Sockel 18 aus Aspekt 1 als auch mit dem Sockel 18 aus Aspekt 2 verwendet werden kann.

Im Speziellen weisen die Behälter aus Fig. 3a bis 3c Vorsprünge oder Wölbungen 22 auf, um eine Stabilität des Behälters 10, 10' beim Abstellen des Behälters 10, 10' auf dessen Ablagefläche zu verringern. Beispielsweise zeigen Fig. 3a und 3c eine Wölbung 22 in der Auflagefläche des Behälters 10, 10' und Fig. 3b zeigt einen Vorsprung 22 als Auflagefläche des Behälters 10, 10'.

Die effektive Aufstands- bzw. Auflagefläche, d. h. der Teil der Wölbung 22 oder des Vorsprungs 22, der beim Abstellen des Behälters 10, 10' auf eine Ablagefläche die Oberfläche der Ablagefläche berührt, ist kleiner als 60% bzw. kleiner als 75% oder kleiner als 90% als eine Querschnittsfläche 24 des Behälters 10, 10'. Als Querschnittsfläche 24 kann beispielsweise die größtmögliche Fläche, die die Oberfläche der Flüssigkeit in dem Behälter 10, 10' annehmen kann oder auch eine mittlere Fläche der Oberfläche der Flüssigkeit in dem Behälter 10, 10' sein.

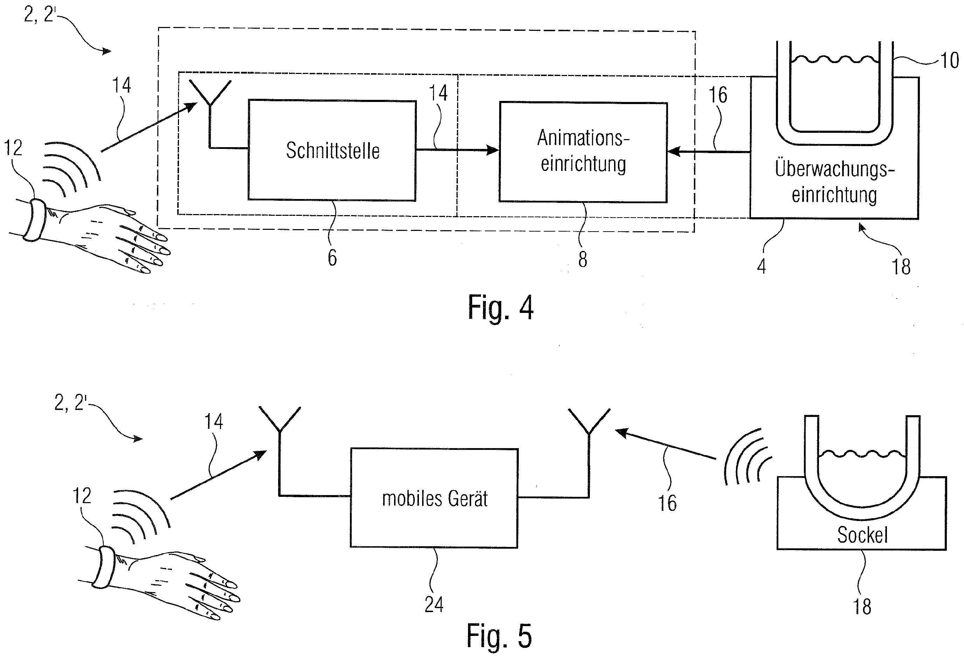

Fig. 4 zeigt das System 2, 2' gemäß einem weiteren Ausführungsbeispiel mit dem Fokus auf der Anordnung der Schnittstelle 6 sowie die Animationseinrichtung 8. So kann die Animationseinrichtung 8 beispielsweise in der Überwachungseinrichtung 4 angeordnet sein. Ergänzend kann auch die Schnittstelle 6 in der Überwachungseinrichtung 4 angeordnet sein, wodurch eine, vorteilhafterweise kabellose, Verbindung zwischen der Animationseinrichtung 8 und der Schnittstelle 6 zu der Überwachungseinrichtung 4 eliminiert bzw. vermieden wird und somit eine potentielle Störung dieser Verbindung vermieden wird. Zudem kann so die Einrichtung des Systems für einen (unerfahrenen) Benutzer erleichtert werden.

Gemäß weiteren Ausführungsbeispielen können jedoch auch die Schnittstelle 6 und die Animationseinrichtung 8 entfernt von der Überwachungseinrichtung 4 und dem Vitalparametersensor 12 angeordnet sein. So ist es beispielsweise möglich, eine Relay-Station für eine Mehrzahl von Vitalparametersensoren 12 und/oder eine Mehrzahl von Überwachungseinrichtungen 4 zu formen, die zwischen dem/den Vitalparametersensor(en) 12 und der/den Überwachungseinrichtung(en) 4 den Datenaustausch vornimmt. So kann ein Benutzer auch mehrere Vitalparametersensoren 12 verwenden, um in Kombination der ermittelten Vitalparameter eine verbesserte Prädiktion bzw. Vorhersage der minimalen bzw. optimalen aufzunehmenden Flüssigkeitsmenge zu ermitteln.

Ferner kann der Benutzer auch parallel mehrere Systeme verwenden, die z. B. über das Relay einen Austausch der ermittelten entnommenen Flüssigkeitsmengen vornehmen, oder dieselben zur Auswertung an das Relay oder einen entfernt angeordneten (zentralen) Server übermitteln. So kann in dem entfernt angeordneten Gerät auch die Auswertung der Vitalparameter und/oder des Füllstands des Behälters 10, 10' erfolgen, wobei die Daten von dem Vitalparametersensor 12 bzw. der Überwachungseinrichtung 4 an das entfernt angeordnete Gerät kabelgebunden oder drahtlos übermittelt werden können.

Die drahtlose Verbindung kann z. B. mittels WLAN, Bluetooth, NFC oder ähnlichen geeigneten Datenübertragungsverfahren erfolgen. In anderen Worten kann die Rechenleistung des Systems zur Auswertung der relevanten Daten (vollständig) in dem entfernt angeordneten Gerät angeordnet sein.

Gemäß weiteren Ausführungsbeispielen kann die Animationseinrichtung 8 ausgebildet sein, um den Nutzer direkt über das entfernt angeordnete Gerät bei Bedarf zum Trinken zu animieren. Es ist jedoch auch möglich, das Ergebnis der Auswertung, also ein Signal, dass der Nutzer zum Trinken animiert werden soll, an die Überwachungseinrichtung 4 bzw. den Behälter 10, 10' oder den Vitalparametersensor 12 zu übermitteln. So kann der Vitalparametersensor 12 beispielsweise ein Display, einen Vibrationsalarm und/oder eine LED oder ein anderes Leuchtmittel aufweisen, um den Nutzer direkt in der unmittelbaren Nähe zum Trinken zu animieren.

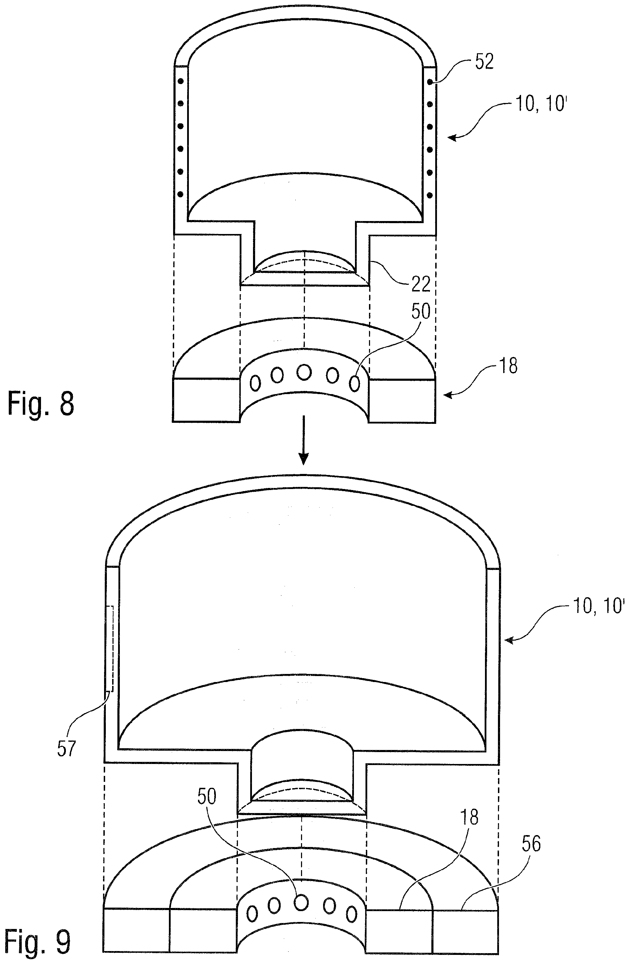

Ferner ist es auch möglich, dass der Behälter 10, 10' die Animationsaufgabe übernimmt. Dies wird nachfolgend beispielsweise mit Bezug auf Fig. 8 näher erläutert werden.

Fig. 5 zeigt das System 2,2' gemäß einem weiteren Ausführungsbeispiel. Hier ist ein mobiles Gerät 24, z. B. ein Transceiver oder ein Mobiltelefon bzw. ein Handy in der Übertragungsstrecke zwischen dem Vitalparametersensor 12 und dem Behälter 10, 10' angeordnet. Hier kann das mobile Gerät 24 z. B. als Relay fungieren, und den Vitalparameter 14 bzw. die entnommene Flüssigkeitsmenge 16 empfangen und optional auswerten.

Die Auswertung kann ferner auch zentral auf einem entfernt angeordneten Server ausgeführt werden.

So kann beispielsweise eine Handy-App (Handy-Applikation) bzw. ein Programm, das auf einem Mobiltelefon 24 ausgeführt wird, die empfangenen Vitalparameter 14 sowie die entnommene Flüssigkeitsmenge 16, z. B. in regelmäßigen Zeitabständen oder bei einer Änderung der Flüssigkeitsmenge empfangen und protokollieren.

Ergänzend oder alternativ können die zu protokollierenden Daten an einen zentral angeordneten Server übermittelt werden. Basierend auf den empfangenen Vitalparametern 14 sowie den entnommenen Flüssigkeitsmengen 16 kann an dem mobilen Gerät 24, an dem Vitalparametersensor 12, an dem Behälter 10, 10' und/oder an dem Sockel 18, in dem der Behälter 10, 10' angeordnet ist, die Aufforderung bzw. Animation des Nutzers zum Trinken erfolgen.

Für alle Ausführungsbeispiele der vorliegenden Offenbarung gilt alternativ oder zusätzlich, dass der Behälter 10, 10' und/oder der Sockel 18 jeweils ausgebildet sein kann, um direkt mit einer Empfangs- und Auswerteinheit, wie beispielsweise einem zentralen Cloud-Server, zu kommunizieren. In anderen Worten könnte bei einem solchen Ausführungsbeispiel auf eine Übermittlung beziehungsweise Weiterleitung von Daten mittels des eingangs erwähnten, als Relay fungierenden, mobilen Geräts 24 verzichtet werden.

Beispielsweise könnten derartige Behälter 10, 10' und/oder Sockel 18 eine integrierte Steuereinheit (z.B. CPU, ASIC, etc.) aufweisen, die in der Lage ist, sich direkt mit einem zentralen Cloud-Server zu konnektieren, zum Beispiel mittels eines GSM-Moduls oder eines frei verfügbaren Netzes wie LORA.

Eine im Behälter 10, 10' und/oder im Sockel 18 integrierte Steuereinheit kann beispielsweise dazu in der Lage sein, direkt programmiert zu werden. Alternativ oder zusätzlich könnte die Steuereinheit ausgebildet sein, um Nutzerdaten, wie zum Beispiel Alter, Größe, Geschlecht usw., zu erfassen und optional eigenständige, darauf basierende, Berechnungen durchführen. Alternativ oder zusätzlich kann die Steuereinheit ausgebildet sein, um Nutzungsdaten an den Server zu übermitteln und optional Firmware-Updates zu akquirieren und einzuspielen.

Somit wäre der Behälter 10, 10' und/oder der Sockel 18 "IoT"-fähig (IoT -engl.: Internet of Things), gegebenenfalls auch ohne Vorhandensein eines zusätzlichen Relays, wie zum Beispiel dem oben erwähnten mobilen Gerät 24.

Unabhängig davon, ob der Behälter 10, 10' und/oder der Sockel 18 nun direkt, oder indirekt mittels eines Relays, mit einer Empfangs- und Auswerteeinheit kommuniziert, kann für die Auswertung bzw. die Bestimmung, ob der Benutzer zum Trinken animiert werden soll, vorteilhafterweise die Historie der aufgezeichneten Daten herangezogen werden, so dass eine Empfehlung, z. B. für den Zeitraum seit dem Aufwachen des Benutzers, ausgegeben wird. Das Aufwachen kann beispielsweise auch durch den Vitalparametersensor 12 bzw. anhand der aufgezeichneten Vitalparameter 14 ermittelt werden.

In anderen Worten kann die Animationseinrichtung 8 ausgebildet sein, um den Behälter 10, 10', den Vitalparametersensor 12 oder das mobile Gerät 24 zu animieren, um den Benutzer visuell bzw. optisch, auditiv bzw. akustisch oder taktil bzw. haptisch zum Trinken zu animieren. Hierfür kann die Animationseinrichtung 8 beispielsweise in dem mobilen Gerät 24 angeordnet sein.

Gemäß weiteren Ausführungsbeispielen kann das System in eine bestehende Gesundheits-App implementiert werden, die optional bereits ausgebildet ist, um Daten von einem Vitalparametersensor 12 zu erhalten. Somit ist eine Ergänzung der App um eine Methode, die eine Auswertung der bereits vorhandenen Vitalparameter 14 basierend auf den zu empfangenen entnommenen Flüssigkeitsmengen 16 durchführt und daraus eine Empfehlung zur Aufnahme der Flüssigkeit ableitet, möglich. Somit kann mit einem geringen Aufwand ein bestehendes System um die Fähigkeit erweitert werden, eine individuelle Trinkempfehlung für den Benutzer auszugeben.

In der Handy-App kann der Nutzer auch persönliche Daten wie das Alter, Geschlecht, Größe, Gewicht, sportliche Aktivität etc. eintragen bzw. erfassen. Diese Parameter können bei der Berechnung der empfohlenen bzw. vorgeschlagenen Trinkmenge mit einbezogen werden. Ergänzend oder alternativ können Geodaten und/oder die Außentemperatur in die Berechnung mit einfließen.

Gemäß Ausführungsbeispielen kann die Handy-App oder auch ein Web-Interface bzw. eine Internet Anwendung anhand dieser persönlichen Daten einen personalisierten Vorschlag für die Flüssigkeitszufuhr erhalten. So haben Menschen in verschiedenen Altersklassen unterschiedliche körperliche Anforderung an die Nahrungs- und Flüssigkeitszufuhr.

Durch die Erfassung des Behältnis-Inhaltes (durch Scannen des GTIN oder Quickscan) und die Bestimmung von z.B. Mineralien, Nährstoffen usw. können dem Nutzer seinen persönlichen Parametern entsprechende Ratschläge zu seinem Trinkverhalten gegeben werden (so sind Flüssigkeiten mit geringem Calcium-Gehalt für ältere Personen nachteilig usw.). Dies könnte z.B. über eine entsprechende Einblendung in der Applikation des Smartphones oder im Rahmen der Web-Applikation erfolgen.

Gemäß weiteren Ausführungsbeispielen hat der Nutzer über die Handy-Applikation jederzeit die Möglichkeit, sich die "Ist-Daten" und die historischen Daten (z.B. 90 Tage) anzeigen zu lassen. Die Applikation kann jedoch jeden Datensatz unverzüglich an eine zentrale Datenbank übermitteln. Diese Datenbank ermöglicht dem Nutzer, sich die Daten in einer Web-Applikation über einen verlängerten Zeitraum (z.B. 360 Tage) anzeigen zu lassen.

Insoweit handelt es sich hier um personalisierte Daten, die entsprechend geschützt (Datenschutz) vorgehalten werden. Alle Daten können entpersonalisiert, d.h. ohne persönliche Informationen, in eine weitere Datenbank übertragen werden um dort empirische Aussagen über das gesamte Nutzerverhalten aller Personen, die das System nutzen, ableiten zu können.

Einen Extrakt hieraus bekommt der Nutzer auf Wunsch mit angezeigt, in dem er sich mit Personen des gleichen Geschlechts, der gleichen Altersgruppe usw. vergleichen kann.

Durch diesen "Gaming-Effekt", also den Vergleich mit anderen Nutzern, ergibt sich der Vorteil, dass der Nutzer mit Personen seiner Vergleichsgruppe quasi in den Wettbewerb tritt und somit "freiwillig" mehr trinkt.

All die beschriebenen Aspekte, die mittels einer Handy-Applikation ausführbar wären, könnten alternativ oder zusätzlich mit der zuvor erwähnten, in dem Behälter 10, 10' und/oder dem Sockel 18 integrierten Steuereinheit, die auch eine kombinierte Steuer- und Kommunikationseinheit sein kann, ausführbar sein.

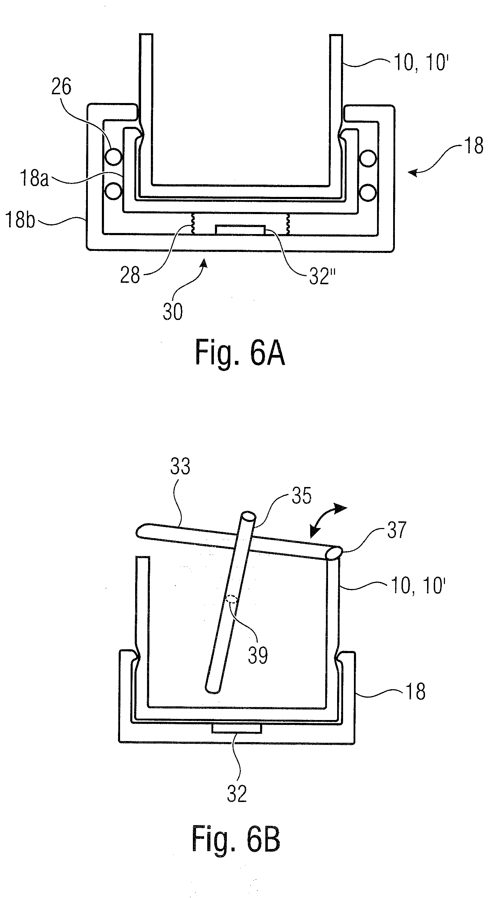

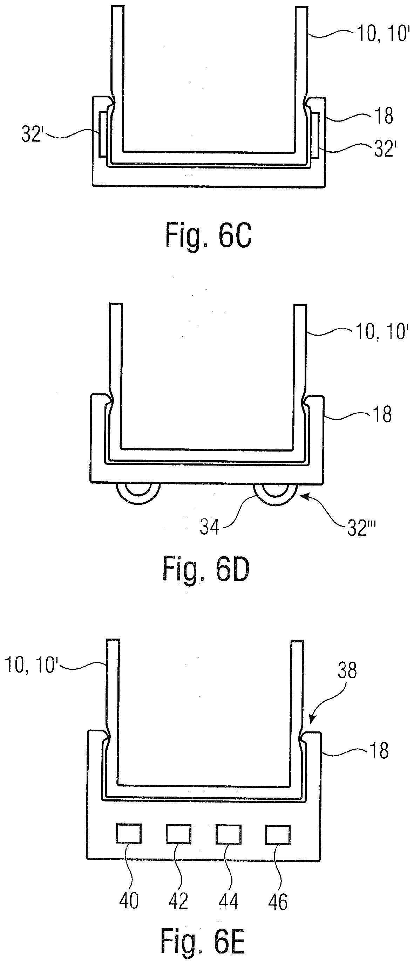

Fig. 6 zeigt eine schematische Querschnittsansicht des Behälters 10, 10' in Verbindung mit dem Sockel 18. In den Fig. 6a bis 6e sind ferner verschiedene Ausführungsformen des Sockels 18 gezeigt.

So werden im Folgenden Ausführungsbeispiele gezeigt, in denen die Überwachungseinrichtung 4 bzw. der Sockel 18 einen Sensor 32 aufweist, der ausgebildet ist, um einen aktuellen Füllstand des Behälters 10, 10' zu ermitteln.

Zum Beispiel anhand einer zeitlichen Folge von ermittelten aktuellen Füllständen des Behälters 10, 10' kann die Überwachungseinrichtung 4 die aus dem Behälter 10, 10' entnommene Flüssigkeitsmenge bestimmen. Hinsichtlich der Fig. 6a-e wird ausgeführt, dass die Überwachungseinrichtung 4 in Ausführungsbeispielen ausgebildet ist, um die aus dem Behälter 10, 10' entnommene Flüssigkeitsmenge (insbesondere) mittels einer kapazitiven Messung, mittels einer optischen Messung, mittels Ultraschall, mittels Radar, mittels einer Gewichtsänderung, mittels einer Laufzeitmessung und/oder mittels einer Leitfähigkeitsmessung zu ermitteln. Somit kann der vorgenannte Sensor 32 ein Sensor sein, der die Messung gemäß dem genannten Verfahren ausführt.

Fig. 6a zeigt den Sockel 18, der ein Aufnahmeelement 18a für den Behälter 10, 10' sowie ein Rahmenelement 18b aufweist, das eine äußere Begrenzung des Sockels 18 formt.

Das Aufnahmeelement 18a kann beispielsweise über Kugellager 26 beweglich in dem Rahmenelement 18b angeordnet sein. Ferner können zwischen dem Aufnahmeelement 18a und dem Rahmenelement 18b Federelemente 28 angeordnet sein, die den Behälter 10, 10' in einem nicht gefüllten bzw. leeren Zustand in einer ersten Position halten, und in einem gefüllten Zustand nachgeben um den Behälter 10, 10' in einer zweiten Position zu halten. In der zweiten Position ist der Behälter 10, 10' demnach näher an einer Aufstellfläche 30 des Sockels 18 angeordnet, als in dem ersten Zustand.

Die Messung des Füllstands einer Flüssigkeit im Behälter 10, 10', die Rückschluss auf die entnommene Flüssigkeitsmenge ermöglicht, kann z. B. über die Gewichtskraft des Behälters 10, 10', z. B. mittels einem oder einer Mehrzahl von an dem Federelement 28 angebrachten Dehnungsmessstreifen, oder kapazitiv über die Änderung des Abstands des Aufnahmeelements 18a zu dem Rahmenelement 18b gemessen werden. Zur kapazitiven Messung kann zwischen dem Aufnahmeelement 18a und dem Rahmenelement 18b eine Elektrode 32'' angeordnet sein, die den Abstand des Aufnahmeelements 18a zum Rahmenelement 18b bestimmt.

Gemäß einem Ausführungsbeispiel kann ergänzend oder alternativ zu dem Federelement 28 auch ein Piezoelement bzw. ein Piezosensor zwischen dem Aufnahmeelement 18a und dem Rahmenelement 18b angeordnet sein, das bzw. der basierend auf seiner Verformung eine Gewichtskraft des Behälters 10, 10' ermitteln kann.

Fig. 6b zeigt den Sockel 18 mit dem Sensor 32, der ausgebildet ist, um vom Boden bzw. ausgehend von einer Aufstandsfläche des Sockels 18, den Füllstand innerhalb des Behälters 10, 10' zu ermitteln. So kann beispielsweise mittels elektromagnetischer Wellen bzw. Strahlung, d. h. z. B. mittels Ultraschall, mittels optischer Strahlung bzw. Licht oder mittels Radar, der Füllstand beispielsweise anhand einer Reflektion an der Grenzschicht zwischen (einer Oberfläche) der Flüssigkeit in dem Behälter zu dem umgebenden Medium, in der Regel Luft, gemessen werden.

Beispielsweise kann über eine Laufzeitmessung zwischen der ausgesendeten elektromagnetischen Strahlung und der empfangenen, reflektierten elektromagnetischen Strahlung ein Rückschluss auf die Füllhöhe des Behälters 10, 10' gezogen werden.

Eine Messmethode von einer Seite des Behälters 10, 10', also z. B. von unten, kann insbesondere in einer hier vorliegenden feuchten Umgebung vorteilhaft sein, da somit die gesamte Elektronik des Systems in einer Platine vorteilhaft im Sockel 18 ausgeführt werden kann. Hierdurch werden, insbesondere in feuchten Umgebungen, fehleranfällige Verbindungen bzw. Kontakte zwischen verschiedenen Platinen oder voneinander entfernten Teilsystemen vermieden. Eine einzelne fest verlötete Platine ohne externe Drähte hat hier im Hinblick auf eine geringe Fehleranfälligkeit Vorteile.

Ferner kann der Behälter 10, 10' auch einen Deckel 33 aufweisen, in dem gemäß Ausführungsbeispielen auch der Sensor 32 bzw. ein Teil des Sensors 32 angeordnet sein können.

So ist es beispielsweise möglich, eine elektromagnetische Welle, beispielsweise einen Lichtstrahl, der vom Übergang der Flüssigkeit in den Behälter 10, 10' in die umgebende Luft gebrochen wird, zu detektieren und anhand der Auslenkung und optional des Einfallswinkels die Füllstandshöhe der Flüssigkeit in dem Behälter 10, 10' zu bestimmen.

Gemäß einem weiteren Ausführungsbeispiel kann auch ein (kapazitiver) Näherungssensor 32 in dem Deckel 33 angeordnet sein, der einen Abstand des Sensors 32 zur Oberfläche der im Behälter 10, 10' vorhandenen Flüssigkeit bestimmen und somit den Füllstand ermitteln.

Ein Sensor 32 in dem Deckel 33 kann vorteilhaft mit einem weiteren Sensor 32 kombiniert werden, der den Verschluss des Deckels 33 anzeigt, so dass diese Messung durchgeführt bzw. ausgewertet wird, wenn der Deckel 33 geschlossen ist.

Der Deckel 33 kann z. B. die Form einer Trinkhilfe aufweisen, sodass der Behälter 10, 10' in Kombination mit dem Deckel 33 eine Schnabeltasse formen kann.

Gemäß Ausführungsbeispielen kann der Deckel 33 für den Behälter 10, 10', also z.B. ein Glas oder eine Karaffe oder eine Flasche, darin enthaltene Flüssigkeiten wie z.B. Leitungswasser reinigen. Der Deckel 33 kann hierfür z.B. eine UV-Lampe aufweisen, die UV-Strahlung aussendet um die Flüssigkeit von darin enthaltenen Keimen zu befreien.

Für eine Energieversorgung des Deckels 33 bzw. der UV-Lampe kann, ähnlich wie bei dem Sockel 18, ein Schwunggenerator oder eine Photovoltaikzelle in bzw. auf dem Deckel 33 angeordnet sein, so dass eine autarke Stromversorgung des Deckels 33 möglich ist.

Alternativ oder ergänzend kann der Deckel 33 auch mit dem Sockel 18 bzw. der Überwachungseinrichtung 4 oder der Animationseinrichtung 8 elektrisch verbunden sein. Eine elektrische Verbindung über eine elektrische Leitung kann z.B. bei einem aufklappbaren Deckel 33, der über ein Scharnier 37 mit dem Behälter 10, 10' verbunden ist, jedoch standardmäßig nicht von diesem getrennt wird, in bzw. über das Scharnier 37 bzw. in unmittelbarer Nachbarschaft des Scharniers 37 ausgeführt sein.

Ferner kann der Deckel 33 eine Steuerung (bzw. Intelligenz) aufweisen, die die UV-Lampe ansteuert um eine dauerhafte UV-Licht Ausstrahlung zu verhindern. So kann die Steuerung die UV-Lampe zyklisch ein- und ausschalten, die UV-Lampe bei geöffnetem Deckel 33 ausschalten oder wie nachfolgend detailliert beschrieben, basierend auf einer Nahrungsmittel- bzw. Flüssigkeitsanalyse, in der eine aktuelle Keimbelastung der Flüssigkeit ermittelt wurde, eingeschaltet werden, wenn die Keimbelastung einen (voreingestellten) Grenzwert überschreitet.

Gemäß weiteren Ausführungsbeispielen kann die Animationseinrichtung 8 auch ergänzend oder alternativ im Deckel 33 ausgeführt sein.

Zusätzlich oder anstelle des Behältnisses 10, 10', hier insbesondere eine Trinkflasche, kann auch der Deckel 33 die Trinkempfehlung z.B. durch Licht signalisieren. Hierbei kann der Deckel 33 entsprechend mit einer kabellosen Datenverbindung mit dem zugehörigen Sockel 18 verbunden sein.

Ferner kann der Deckel 33 eine Durchführung für z.B. einen Strohhalm oder eine sonstige Trinkhilfe 35 aufweisen, bei dem die Trinkmenge über den Durchfluss gemessen bzw. verifiziert werden kann. Die Trinkhilfe 35 kann den ermittelten Durchfluss drahtlos oder durch einen Kontakt mit dem Deckel 33 zur Auswertung der Überwachungseinrichtung 4 bereitstellen. Über die gleiche Verbindung kann auch eine Stromversorgung hergestellt werden. Der Durchfluss kann über einen in der Trinkhilfe angeordneten Durchflusssensor 39 ermittelt werden. Ein Deckel 33 mit und ohne Trinkvorrichtung 35 ist auch für ein Trinkglas verwendbar, wobei hier das Glas z.B. für "to go"-Getränke, also Getränke zum Mitnehmen, Verwendung finden kann.

In anderen Worten kann das System eine Trinkhilfe 35 aufweisen, wobei die Trinkhilfe 35 ausgebildet ist, um einen Durchfluss einer dem Behälter 10, 10' durch die Trinkhilfe 35 entnommenen Flüssigkeitsmenge zu bestimmen und die dem Behälter 10, 10' durch die Trinkhilfe 35 entnommenen Flüssigkeitsmenge der Überwachungseinrichtung 4 bereitzustellen.

Die Überwachungseinrichtung 4 kann die dem Behälter 10, 10' durch die Trinkhilfe 35 entnommenen Flüssigkeitsmenge mit der durch eines der weiteren beschriebenen Verfahren ermittelte entnommene Flüssigkeitsmenge des Behälters 10, 10' vergleichen um eine optimierte entnommene Flüssigkeitsmenge zu bestimmen. So können kleinere Ungenauigkeiten bei der Bestimmung der entnommenen Flüssigkeitsmenge z.B. durch Mittelung minimiert werden und es kann ferner, bei einer Abweichung die größer ist als eine typische Toleranz der Messverfahren, eine anderweitige Entnahme von Flüssigkeit beispielsweise durch Auskippen des Inhalts des Behälters 10, 10' festgestellt werden.

Ergänzend oder alternativ kann die dem Behälter 10, 10' durch die Trinkhilfe 35 entnommene Flüssigkeitsmenge auch als Referenz, also als alleinige entnommene Flüssigkeitsmenge zur Auswertung in der Überwachungseinrichtung 4 herangezogen werden. Beispielsweise in Kombination mit einem fest aufsitzenden Deckel 33, der nicht dafür vorgesehen ist, beim Trinken abgenommen zu werden, kann die dem Behälter 10, 10' durch die Trinkhilfe 35 entnommenen Flüssigkeitsmenge der von dem Benutzer aufgenommenen Flüssigkeitsmenge am genauesten entsprechen.

Fig. 6c zeigt den Sockel 18 mit Sensoren 32', die in dem gezeigten Ausführungsbeispiel an den Seitenwänden des Behälters 10, 10' bzw. des Sockels 18 platziert sind.

Vorteilhafterweise werden die Sensoren 32' hier vollständig über eine komplette Höhe des Behälters 10, 10' angeordnet, so dass ausgesendete elektromagnetische Strahlung bzw. Signale eines Sensorelements 32' an dem gegenüberliegenden Sensorelement 32' empfangen werden können.

Liegt die Oberfläche bzw. die Grenzschicht zwischen der Flüssigkeit und der umgebenden Luft innerhalb der Detektorfläche, kann über die Laufzeitunterschiede des ausgesendeten Signals durch die Luft und in der Flüssigkeit die aktuelle Füllstandshöhe der Flüssigkeit in dem Behälter 10, 10' ermittelt werden.

Fig. 6d zeigt den Sockel 18 mit Aufstandselementen 34, beispielsweise Füßen oder Noppen, die unterhalb der Aufstandsfläche des Sockels 18 angeordnet sein können.

In den Aufstandselementen 34 kann, ähnlich wie in Fig. 6a bereits gezeigt, eine Messung des Inhalts des Behälters 10, 10' über eine Gewichtsänderung des Behälters 10, 10' erfolgen, die mittels des Sensors 32''', der in einem oder mehreren Aufstandselementen 34 angeordnet ist, gemessen werden.

Der Sensor 32''' ist beispielsweise ein Dehnungsmessstreifen, ein Piezoelement oder ein kapazitiver Sensor. Dies ist vorteilhaft, da die Aufstandselemente 34 beispielsweise mittels einer lösbaren Verbindung, wie einer Schraub- oder Steckverbindung, mit dem Sockel 18 verbunden sein können, wodurch der Gewichtssensor 32''' bei einem Defekt kostengünstig ausgetauscht werden kann.

Fig. 6e zeigt den Sockel 18 mit dem Behälter 10, 10' gemäß einem weiteren Ausführungsbeispiel, wobei die nachfolgend beschriebenen Elemente bzw. Merkmale einzeln oder in einer beliebigen Kombination in dem Sockel 18 angeordnet sein können.

So zeigt Fig. 6e einen Befestigungsmechanismus 38, der ausgebildet ist, um den Behälter 10, 10' in einem Betriebszustand mechanisch oder magnetisch fest mit dem Sockel 18 zu verbinden.

Ferner kann der Behälter 10, 10' auch mittels anderer (physikalischer) Kräfte fest, aber nicht untrennbar mit dem Sockel 18 verbunden sein. So ist es vorteilhaft, dass die Verbindung derart fest ist, dass ein Anheben oder eine Bewegung des Behälters 10, 10' in gleichem Maße in einem Anheben bzw. einer Bewegung des Sockels 18 resultiert. Dennoch soll es möglich sein, den Behälter 10, 10' und den Sockel 18, beispielsweise zum Reinigen oder zum Aufbewahren, voneinander zu trennen. Daher kann die Verbindung auch verschraubt, geklemmt oder mittels Pressfitting (dt.: Einpressen) ausgeführt sein.

Ferner kann der Sockel 18 eine Detektionseinheit 40 aufweisen, die ausgebildet ist, um den Behälter 10, 10' in einem Betriebszustand zu identifizieren und gegenüber weiteren Behältern 10, 10' mit jeweils einer spezifischen Füllmenge zu unterscheiden.

Die Unterscheidung der Detektionseinheit 40 ermöglicht es der Überwachungseinrichtung 4, eine behälterspezifische entnommene bzw. getrunkene Flüssigkeitsmenge zu bestimmen und den Behälter 10, 10' dem zugehörigen Benutzer zuzuordnen. Dies ist vorteilhaft, da somit die Sockel 18 universell für jeden Behälter 10, 10' einsetzbar sind und somit ein absichtlicher oder versehentlicher Austausch von benutzten Behältern 10, 10' und zugehörigen Sockeln 18 dennoch die gleichen Ergebnisse für den Benutzer des Behälters 10, 10' liefern.

Zur Erkennung kann der Behälter 10, 10' z. B. einen spezifischen Code, z. B. in Form eines RFID Tags oder eines QR-Codes, aufweisen, um der Detektionseinheit 40 die Ermittlung des aktuellen Behälters 10, 10' zu ermöglichen.

Ferner ist es dann vorteilhaft, auch zugehörige Vitalparameter benutzerspezifisch zu ermitteln, so dass auch eine Vitalparameter-abhängige Bestimmung, ob der Benutzer Flüssigkeit aufnehmen soll, möglich ist.

Ferner kann der Sockel 18 eine Temperatur der Flüssigkeit in dem Behälter 10, 10' in Ausführungsbeispielen erfassen und einstellen. Hierfür kann der Sockel 18 einen Temperaturregler 42 aufweisen, der ausgebildet ist, um eine Temperatur der Flüssigkeit zu ermitteln und bei einer Abweichung von einem beispielsweise durch den Benutzer eingestellten Referenzwert, die Temperatur der Flüssigkeit auf den eingestellten Wert zu erwärmen oder auch abzukühlen.

Das Erwärmen und Abkühlen bzw. die Messung kann auch unter Einstellung einer Hysterese erfolgen. Somit kann der Sockel 18 beispielsweise im Sommer für kühle Getränke und im Winter für eine angenehme Temperierung eines warmen Tees oder anderen Heißgetränks sorgen.

Gemäß weiteren Ausführungsbeispielen kann das System, z. B. in dem Sockel 18, eine Energieversorgungseinheit 44 aufweisen, die ausgebildet ist, um das System 2, 2' mittels Solar, mittels Energierückgewinnung (Energy-Harvesting), mittels eines Generators und/oder eines Schwunggenerators (autark) mit Energie zu versorgen.

In anderen Worten kann das System bzw. der Sockel 18 frei von einer Stromquelle oder zumindest mit einer reduzierten Stromaufnahme betrieben werden, wenn die vorgenannten Mittel zur Energiegewinnung eingesetzt werden. Um eine dauerhafte Energieversorgung zu gewährleisten und Spitzen sowie Senken in der Energieversorgung auszugleichen, kann die Energieversorgungseinheit ferner einen Akkumulator, einen Kondensator oder einen weiteren Energiespeicher aufweisen, der die Energie speichert bzw. puffert.

Beispielsweise für kleinere oder preiswertere Systeme kann jedoch auch eine externe Stromversorgung, z. B. mittels eines USB-Anschlusses oder mittels Induktion (z.B. über near-field communication (NFC), dt.: Nahfeldkommunikation), dauerhaft oder zum Laden des Energiespeichers vorgesehen werden. Ein autarkes oder zumindest teilweise autarkes System ist jedoch im Hinblick auf ökologische Aspekte vorteilhaft.

Die Ladung der Batterie bzw. des Akkumulators kann auch mittels RFID (radio-frequency identification dt.: Identifizierung mit Hilfe elektromagnetischer Wellen), NFC (near-field communication dt.: Nahfeldkommunikation) oder USB (universal serial bus, dt.; universelles, serielles Bussystem) erfolgen. Diese Energieversorgung kann auch zur Kühlung oder Erwärmung des Getränks, z. B. durch ein Peltier-Element, herangezogen werden. Aufgrund des potentiell hohen Energieverbrauchs des Heiz- bzw. Kühlelements, z. B. wenn große Temperaturunterschiede überwunden werden sollen, kann auch eine separate Stromversorgung hierfür vorgesehen sein.

Weitere Ausführungsbeispiele zeigen das System zur Verwendung in der Gastronomie.

Hier kann, wenn der Füllstand des Glases 10, 10' einen vordefinierten Schwellenwert unterschreitet, die Servicekraft bzw. der Wirt hierüber informiert werden. Diese Erkennung, dass der Füllstand des Glases 10, 10' den vordefinierten Schwellenwert unterschritten hat, kann mittels der Überwachungseinrichtung 4 erfolgen. Die Signalisierung mittels der Animationseinrichtung 8 kann dann dezentral z.B. an einer zentralen Aufnahme, wie einem Serviceterminal für die Servicekräfte, durch eine entsprechenden Meldung des Glases 10, 10' bzw. des Sockels 18 erfolgen.

In anderen Worten kann die Animationseinrichtung 8 hier in dem Serviceterminal angeordnet sein und basierend auf dem Ergebnis der Überwachungseinrichtung 4 auf dem Serviceterminal eine Meldung ausgeben. Hier kann die Servicekraft oder der Ausschank das Glas (Behälter) 10, 10' bzw. den Sockel 18 z.B. mit Tischnummer und Glasinhalt definieren bzw. eindeutig zuordnen. Der Gastraum oder auch der Biergarten sollten dann mit entsprechenden Receivern bzw. Empfängern ausgestattet sein, die eine entsprechende Meldung an den Wirt, den Ausschank, die Servicekraft oder ein zentrales Tableau bzw. Terminal übermittelt.

Ergänzend oder alternativ kann die Servicekraft gemäß einem Ausführungsbeispiel mit einem Armband ausgestattet werden, durch das sie z.B. durch Vibration darüber informiert wird, dass sie sich in der Nähe eines leeren oder fast leeren Glases 10, 10' befindet. Hier kann die Animationseinrichtung 8 in dem Armband angeordnet sein. Das Armband der Servicekraft kann hier beispielsweise einen vorgegebenen Radius von z.B. fünf Metern ableuchten bzw. Signale des Behälters/Sockels in diesem Radius empfangen und bei Erhalt eines Signals der Überwachungseinrichtung eine entsprechende Signalisierung an dem Armband auslösen.

Beide vorgenannten Ausführungsbeispiele haben einen Vorteil für den Gast, dass er nicht verdurstet bzw. durch den Service gerne länger in der Gaststätte verweilt, den Gastwirt, da ggf. mehr Getränke umgesetzt werden oder weitere Produkte wie z.B. Dessert usw. konsumiert werden sowie die Servicekraft, die durch Umsatzanteil oder einem höheren Trinkgeld partizipiert und schneller auf die Wünsche des Gastes reagieren kann.

Die Gläser 10, 10' können für beide Verfahren mit einem RFID- oder NFC-Chip ausgestattet sein, der entweder innerhalb oder außerhalb des Glases 10, 10' aufgebracht werden kann. Somit kann die Überwachungseinrichtung 4 mit der Animationseinrichtung 8 kommunizieren, d.h. ein Signal übermitteln, dass der Flüssigkeitsstand in dem Glas 10, 10' den voreingestellten Schwellenwert unterschritten hat. Dieser Chip kann der gleiche RFID- oder NFC-Chip sein, der wie im Vorhinein beschrieben auch zum Laden des Energiespeichers in dem Sockel 18 bzw. Behälter 10, 10' verwendet wird.

Gemäß weiteren Ausführungsbeispielen kann der Sockel 18 einen Neigungssensor 46 aufweisen, der ausgebildet ist, um einen Neigungswinkel des Behälters 10, 10' zu erfassen. Mittels des Neigungssensors 46 kann verifiziert werden, ob aktuell aus dem Behälter 10, 10' getrunken wird. In den Momenten, in denen aus den Behältern 10, 10' getrunken wird, kann eine verlässliche Messung des Füllstands des Behälters 10, 10' gestört sein, was bei der Messung berücksichtigt werden sollte.

Ferner können über einen Abgleich der gemessenen Füllstandsmenge mit dem Neigungssensor 46 kleinere Abweichungen in der aktuellen Füllstandsmenge gemittelt bzw. ausgeglichen werden.

Ebenso kann der Neigungssensor 46 wesentlich zur Energieeinsparung des Systems beitragen, indem eine Füllstandsmessung beispielsweise zu Beginn einer einsetzenden Neigung und nach Abstellen des Behälters 10, 10', d. h. nach einer Rückführung aus der Neigung, gemessen wird. Aus der Differenz kann dann effizient die getrunkene Flüssigkeitsmenge ermittelt werden. Wird anhand des Neigungssensors 46 keine Neigung des Systems erkannt, können alle Sensoren und nicht benutzten Stromverbraucher abgeschaltet oder in den Stand-By-Modus versetzt werden, um eine möglichst geringe Energieaufnahme des Sockels 18 zu ermöglichen.

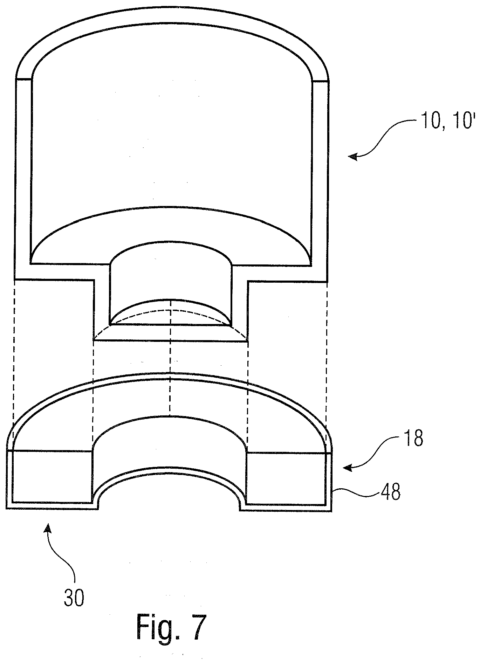

Fig. 7 zeigt den Behälter 10, 10' sowie den Sockel 18 gemäß einem weiteren Ausführungsbeispiel. Gemäß diesem Ausführungsbeispiel weist der Sockel 18 eine Ummantelung 48 auf, die ausgebildet ist, um den Sockel 18 vor äußeren Einflüssen, beispielsweise Umwelteinflüssen, zu schützen oder eine rutschhemmende Aufstandsfläche 30 zu formen. Dies ist vorteilhaft, da der Sockel 18 so beispielsweise vor auslaufenden Flüssigkeiten bzw. auch beim Reinigen vor Reinigungsflüssigkeiten geschützt werden kann.

Hierfür kann die Ummantelung 48, wie in Fig. 7 gezeigt, die Fläche, die nicht von dem Behälter 10, 10' bedeckt wird, einschließen oder auch eine vollständige Ummantelung des Sockels 18 (nicht gezeigt) ermöglichen.

So kann die Ummantelung 48 auch den hinsichtlich Fig. 6e gezeigten Befestigungsmechanismus 38 formen, indem ein rutschhemmendes Material zwischen einer Verbindungsstelle des Sockels 18 und des Behälters 10, 10' in Form der Ummantelung 48 eingebracht wird.

Dieses oder ein anderes rutschhemmendes Material kann auch auf der Unterseite des Sockels 18 aufgebracht sein, um ein leichtes Verrutschen auf einer glatten Oberfläche zu vermeiden und dem Sockel 18 und dem Behälter 10, 10' einen sichereren Stand zu ermöglichen. Der Sockel 18 kann somit rutschsicher, kippsicher und/oder bruchsicher sein.

Ein Material für die Ummantelung 48 kann Silikon aufweisen. Die Ummantelung 48, z. B. ein Silikoncover kann die Bodengruppe bzw. den Sockel 18 vor Flüssigkeiten, Stößen, und Erschütterungen schützen. Ferner kann es, beispielsweise analog zu bekannten Handy-Hüllen, zur Individualisierung des Sockels 18 beitragen, indem austauschbare Cover 48 mit unterschiedlichen Farben, Formen oder Mustern angeboten und vertrieben werden.

Ferner besteht die Möglichkeit der Individualisierung z. B. mittels Gravuren, Verzierungen, Farbe, Form, Material, Prägungen, Überzug an Sockel (Ummantelung), Clips, etc.

Der Unterbau bzw. der Sockel 18 kann, z. B. durch die Ummantelung 48 spritzwassergeschützt oder gemäß der Schutzarten IP55/IP57 bzw. IP 67/69 geschützt sein und somit auch spülmaschinenfest gegenüber Feuchtigkeit und auch gegenüber der Temperatur sein.

Eine Gravur oder eine Laserverzierung in dem Behälter 10, 10' bzw. in einer Seitenwand des Behälters 10, 10' kann auch das Animieren des Benutzers zum Trinken verstärken.

So kann durch die spezielle Einstreuung des Lichtes durch den Sockel 18 bzw. die Animationseinrichtung in das Glas 10, 10', wobei das Glas 10, 10' zu einem Lichtleiter wird, die Gravur oder auch das Lasern vorteilhaft sein, da die Gravur bzw. die Laserung zu einer Bruchkante im Glas 10, 10' führt, an der sich das Licht (in besonderer Weise) bricht. Durch das eingestreute Licht können somit besondere Effekte erzielt werden, die die Animationswirkung, z.B. für Kinder, verstärken.

Der Sockel 18 kann ferner einen Erkennungsmechanismus aufweisen, der ein auf den Kopf kippen bzw. stellen des Sockels 18 mit verbundenem Behälter 10, 10' erkennt und eine entsprechende Warnung z.B. in Form eines (roten) Leuchtens, einer Vibration und/oder eines Warntons ausgibt um einen typischerweise unerwünschten Zustand zu signalisieren.

Das auf den Kopf kippen kann bei einem beliebigen Neigungswinkel eintreten, wobei beispielhaft Neigungswinkel von mehr als 130°, mehr als 145° oder mehr als 160° möglich sind.

Der Neigungswinkel kann als Drehung des Systems, also hier der Kombination aus Behälter 10, 10' und Sockel 18 um eine beliebige Achse des Systems ausgehend von einem Grundzustand angesehen werden, wobei der Grundzustand z.B. das auf dem Sockel 18 abgestellte System kennzeichnet. Somit kann vor dem Abstellen des Behälters 10, 10' in Verbindung mit dem Sockel 18 (kopfüber) im Schrank gewarnt werden um einer unnötigen Entladung des Sockels 18 im angeschlossenen Zustand vorzubeugen.

Ferner kann z.B. bei einem nicht spülmaschinenfest ausgeführten Sockel 18 vor dem Abstellen des Behälters 10, 10' mit dem Sockel 18 in der Spülmaschine gewarnt werden, wenn der Benutzer vergessen hat den Sockel 18 vor dem Einräumen des Behälters 10, 10' in die Spülmaschine zu entfernen. Somit kann einem Schaden des Sockels 18 vorgebeugt werden.

In anderen Worten kann die Animationseinrichtung ausgebildet sein, um bei einem Überschreiten eines Neigungswinkels, der ein auf den Kopf Stellen des Systems repräsentiert, ein Warnsignal auszugeben.

Die Figuren 8 und 9 zeigen eine Querschnittsansicht des Behälters 10, 10' und des Sockels 18 in unterschiedlichen Größen, gemäß einem weiteren Ausführungsbeispiel. In dem gezeigten Ausführungsbeispiel kann der Behälter 10, 10' aus einem lichtdurchlässigen Material gebildet sein.

Die Auswerteeinheit kann ferner zum Animieren Licht, beispielsweise über die Lichtquellen 50 in dem Sockel 18, in den Behälter 10, 10' einkoppeln.

Ferner kann der Behälter 10, 10' Streuzentren 52 aufweisen, die ausgebildet sind, um das eingekoppelte Licht zu streuen.

Gemäß einem weiteren Ausführungsbeispiel kann die Auswerteeinheit ausgebildet sein, um zum Animieren eine elektrische Spannung oder einen elektrischen Strom an den Behälter 10, 10' anzulegen und in dem Behälter 10, 10' eingebrachte oder auf dem Behälter 10, 10' aufgebrachte Animationsmittel anzuregen. Diese Animationsmittel können beispielsweise ein Motor mit einer Unwucht, analog z. B. bei einem Vibrationsalarm eines Mobiltelefons, oder auch in den Behälter 10, 10' eingebrachte Leuchtdioden oder andere Leuchtmittel sein.

Ferner kann auf dem Behälter 10, 10' eine anregbare Farbe aufgebracht sein, die durch Anlegen einer elektrischen Spannung oder eines elektrischen Stroms luminesziert oder fluoresziert und somit Licht aussendet.

Gemäß Ausführungsbeispielen kann die Auswerteeinheit ausgebildet sein, um das Licht an einem Vorsprung 22 in den Behälter 10, 10' einzukoppeln. Dies ist vorteilhaft, da somit durch die Ausbildung des Vorsprungs 22 eine verringerte Aufstellfläche und somit eine Instabilität des Behälters 10, 10' erzeugt wird, die durch eine an den Vorsprung 22 angepasste Aussparung in dem Sockel 18 kompensiert wird. So kann der Behälter 10, 10' in Kombination mit dem Sockel 18 ohne Einschränkungen auf einer Oberfläche stehen, wobei der Behälter 10, 10' ohne den Sockel 18 eine verringerte Standfestigkeit aufweist.

In anderen Worten kann der Behälter 10, 10' in der Formgebung ausgeführt sein, dass derselbe zur Verwahrung abgestellt werden kann. Befüllt man den Behälter 10, 10' jedoch ohne das vorherige Anbringen einer Bodengruppe bzw. des Sockels 18, neigt der Behälter 10, 10' dazu zu kippen.

Wie in Figur 9 abgebildet, kann um den Sockel 18 ein Erweiterungselement 56 angeordnet werden, so dass auch Behälter 10, 10' mit einem vergrößerten Durchmesser, wie z. B. eine Flasche oder eine Karaffe, sicher in dem Sockel 18 platziert werden können. Somit ist eine kostengünstige, da modulare Anordnung geschaffen, um Sockel 18 mit geringem Aufwand an verschiedene Behältergrößen anzupassen.

Ferner zeigen Ausführungsbeispiele, dass eine Karaffe bzw. ein Sockel 18, der an einer Karaffe angebracht ist, einen Sensor aufweist, der beispielsweise in Kombination mit dem Füllstandssensor, der einen Behälter 10, 10' überwacht, erkennt, in welchen Behälter 10, 10' die Flüssigkeit aus der Karaffe eingeschenkt wird und eine entsprechende Personenzuordnung vornimmt. Dieser Wert kann als alleinige Referenz für die eingeschenkte Flüssigkeitsmenge dienen oder aber in Kombination mit einer Auswertung der Füllstandsmessung an dem Behälter 10, 10' eine optimierte Berechnung der im Behälter 10, 10' vorhandenen Flüssigkeitsmenge, z.B. durch Mittelung der Werte oder zur Erkennung von Ausreißern bzw. zur Fehldetektionen herangezogen werden.

Gemäß einem weiteren Ausführungsbeispiel kann der Behälter 10, 10' einen Handsensor 57 aufweisen, der ausgebildet ist, um einen Körperkontakt mit dem Behälter 10, 10' zu erfassen. Der Handsensor 57 ist beispielsweise als kapazitiver Sensor oder als Drucksensor ausgebildet, der einen Körperkontakt mit dem Behälter 10, 10' erfasst. Hierfür können Sensoren 57 verwendet werden, die auch in berührungsempfindlichen Bildschirmen beispielsweise von Smartphones eingesetzt werden.

In anderen Worten kann das Trinkbehältnis 10, 10' mit einer Schicht versehen sein, die eine sensitive kapazitive Berührungsmessung erlaubt. Alternativ oder ergänzend kann der Handsensor 57, wie in Fig. 8 gezeigt, auch in der Wand des Behälters 10, 10' angeordnet sein.

Der Handsensor 57 kann verwendet werden, um Fehlerquellen oder Messlücken zu vermeiden. Eine permanente Überwachung bzw. Messung des Gefäßinhaltes kann sehr energieverbrauchend sein. Über diesen Weg soll es ermöglicht werden, erforderliche Erhebungen dann vorzunehmen wenn das Glas 10, 10' umschließend berührt wird und somit mit einer Flüssigkeitsaufnahme oder einem Nachschenken gerechnet werden kann.

Ebenso kann über den Handsensor 57 zwischen einer absichtlichen und einer unabsichtlichen Flüssigkeitsentnahme unterschieden werden, da beim versehentlichen Umstoßen des Behälters 10, 10' typischerweise kein (durchgehender) Kontakt mit dem Behälter 10, 10' besteht. Es kann somit darauf geschlossen werden, dass die Flüssigkeit aus dem Behälter 10, 10' entnommen jedoch von dem Benutzer nicht aufgenommen bzw. getrunken wurde.

Gemäß weiteren Ausführungsbeispielen kann der Behälter 10, 10', der nicht notwendigerweise einen Sockel 18 aufzuweisen braucht und beispielsweise auch ein Teller sein kann, ein Element zur Analyse der auf bzw. in dem Behälter 10, 10' vorliegenden Nahrungsmittel aufweisen. Dieses Element ist beispielsweise eine Elektrode, die das Nahrungsmittel z. B. mittels Cyclovoltammetrie (bzw. zyklischer Voltametrie oder der Dreiecksspannungsmethode) analysiert. Ferner sind auch andere, z. B. chemische oder physische Analyseverfahren, separat oder in einer Kombination von verschiedenen Verfahren einsetzbar.

Durch die Bestimmung der zugeführten Nahrungsmittel kann z. B. auch eine Nährstoffanalyse durchgeführt werden, so dass die bereits beschriebe Animation zur Aufnahme von Flüssigkeiten dahin gehend erweitert werden kann, dass generell eine Empfehlung zur Aufnahme von Nahrungsmitteln abgegeben werden kann, um eine ausgewogene Ernährung des Benutzers zu unterstützen.

Gemäß einem weiteren Ausführungsbeispiel kann der Nutzer des Systems die eingefüllte Flüssigkeit selbst erfassen. Hierbei genügt es, den EAN/GTIN-Code (EAN: European Article Number, dt.: europäische Artikelnummer; GTIN: Global Trade Item Number, dt.: globale Artikelidentifikationsnummer) von der Getränkeflasche beispielsweise per Smartphone-Kamera zu scannen. Über eine Datenbankabfrage wird ermittelt um was für ein Getränk es sich handelt. Hierfür erforderliche Datenbanken sind frei verfügbar. Über weitere Datenbanken, wie z.B. www.fddb.info, können dann Informationen (Nährwerte, Mineralien usw.) über das jeweilige Getränk abgefragt und in der Applikation hinterlegt werden. Diese Informationen können in einem späteren Schritt, z.B. bei der Analyse des Trinkverhaltens, ausgewertet werden.

Über die Technik im Unterbau bzw. im Sockel 18 wird bei der Erfassung via beschriebenem Codescan ein Quickscan bzw. eine schnelle Analyse des Gefäßinhaltes angestoßen. Dies ermöglicht zum Einen, dass wiederkehrende Getränke vom System sofort wiedererkannt und erfasst werden. Zum anderen kann der Nutzer durch einen Warnhinweis zum neuerlichen Scan des EAN/GTIN-Codes animiert werden, wenn der Gefäßinhalt sich ändert und durch den Nutzer keine Änderung des Getränks in der Applikation vorgenommen wurde (z.B. Wechsel von Fruchtsaft auf Wasser). Dies führt dazu, dass hier nicht versehentlich die falschen Werte in die Analyse des Trinkverhaltens einbezogen werden.

Getränke, die keinem eindeutigen EAN/GTIN-Code zugeordnet werden können (z.B. (selbst gemischte) Mischgetränke wie Schorle usw.), können manuell im System erfasst werden. Hier können jedoch die Teilmengen des Mischgetränks erfasst werden, wenn die zu mischenden Flüssigkeiten nacheinander in den Behälter 10, 10' eingefüllt werden. Somit kann beispielsweise die Auswerteeinheit die genaue Zusammensetzung des Mischgetränks anhand der bekannten Teilmengen der Flüssigkeiten bestimmt werden.

Ferner kann die Nahrungsmittelanalyse auch in Kombination mit den beschriebenen Verfahren zur Füllstandsmessung bzw. zur entnommenen Flüssigkeitsmenge eingesetzt werden. So wird z. B. die in dem Behälter 10, 10' vorhandene Flüssigkeit analysiert, um beispielsweise einen Brechungsindex an der Grenzfläche zwischen der Flüssigkeit und der umgebenden Luft zu bestimmen und somit eines der beschriebenen Verfahren, die auf der Auslenkung einer elektromagnetischen Strahlung beim Ein- bzw. Austritt in bzw. aus der Flüssigkeit auftritt (vgl. z. B. Fig. 6), zu optimieren. Der Brechungsindex kann z. B. basierend auf einer Analyse der Dichte der Flüssigkeit oder basierend auf der Bestimmung der Flüssigkeit über eine vordefinierte hinterlegte Tabelle ermittelt werden.

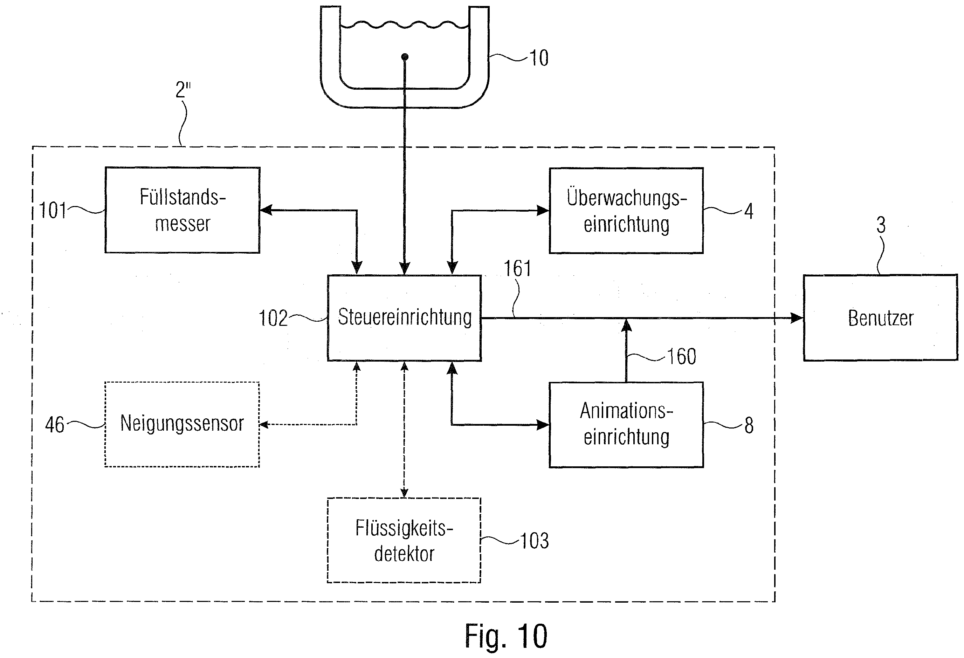

Eine Füllstandsmessung kann beispielsweise mit einem erfindungsgemäßen Füllstandsmesser 101 durchgeführt werden. So zeigt Figur 10 ein Ausführungsbeispiel für ein System 2" gemäß eines weiteren Aspekts dieser Erfindung.

Das System 2" weist unter anderem einen Behälter 10 zum Aufnehmen von Flüssigkeit auf. Ferner weist das System 2", ebenso wie die zuvor beschriebenen Systeme 2, 2', eine Überwachungseinrichtung 4 sowie eine Animationseinrichtung 8 auf.

Wie bereits zuvor beschrieben wurde, ist auch bei dieser Ausführungsform die Überwachungseinrichtung 4 ausgebildet, um eine aus dem Behälter 10 entnommene Flüssigkeitsmenge zu bestimmen, und die Animationseinrichtung 8 ist ausgebildet, um abhängig von der entnommenen Flüssigkeitsmenge den Benutzer 3 zum Trinken zu animieren.

Zusätzlich weist das System 2" gemäß diesem Aspekt einen Füllstandsmesser 101 zum Messen des Füllstands der in dem Behälter 10 befindlichen Flüssigkeit auf. Generell ist das in Figur 10 abgebildete System 2" mit allen zuvor beschriebenen Merkmalen der Systeme 2, 2' kombinierbar.

Wie nachfolgend detaillierter beschrieben wird, kann das System 2" ferner optional einen Flüssigkeitsdetektor 103, optional einen Neigungssensor 46, sowie optional eine Steuereinrichtung 102 aufweisen. Diese Steuereinrichtung 102 kann beispielsweise ein geeigneter IC (engl.: Integrated Circuit), FPGA oder Mikroprozessor beziehungsweise Mikrocontroller sein.

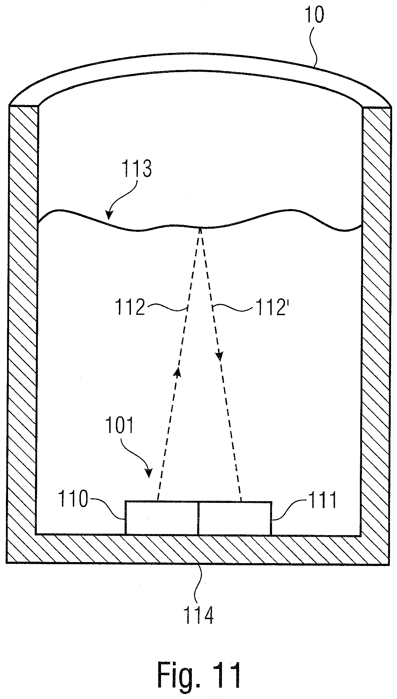

Figur 11 zeigt ein Ausführungsbeispiel eines Behälters 10 für ein erfindungsgemäßes System 2" mit einem Füllstandsmesser 101, wobei der Füllstandsmesser 101 ein optischer Füllstandsmesser ist, der mindestens einen Emitter 110 zum Emittieren von elektromagnetischer Strahlung 112 und mindestens einen Empfänger 111 zum Empfangen der emittierten elektromagnetischen Strahlung 112 aufweist.

Die elektromagnetische Strahlung 112 kann beispielsweise sichtbares Licht im Wellenlängenbereich von 400 nm bis 700 nm sein. Es ist aber ebenso denkbar, dass es sich um UV-Licht im Wellenlängenbereich von 10 nm bis 400 nm, oder um Infrarotlicht im Wellenlängenbereich von 700 nm bis 1000 nm handelt. Ebenso denkbar wäre es, dass es sich bei der elektromagnetischen Strahlung um Schallwellen, zum Beispiel Ultraschall, handelt.

Gemäß eines Ausführungsbeispiels der Erfindung kann der Emitter 110 mindestens einen Laser aufweisen. Beispielsweise kann der Emitter 110 einen Mikrowellenlaser aufweisen. Mikrowellenlaser werden auch als Maser bezeichnet und können elektromagnetische Strahlung im Wellenlängenbereich von 1 mm bis 300 mm aussenden.

Wie in Figur 11 zu erkennen ist, kann der Emitter 110 die elektromagnetische Strahlung 112 beispielsweise gebündelt, zum Beispiel in Form eines Strahls, emittieren. Dies ist einfach und kostengünstig mit den eben erwähnten Lasern möglich.

Gemäß einer denkbaren Ausgestaltung der Erfindung ist der Emitter 110 derart an dem Behälter 10 angeordnet, dass die emittierte elektromagnetische Strahlung 112 auf die zur Umgebung angrenzende Flüssigkeitsoberfläche 113 der in dem Behälter 10 befindlichen Flüssigkeit auftrifft.

Der Empfänger 111 wiederum kann derart an dem Behälter 10 angeordnet sein, dass ein an der Flüssigkeitsoberfläche 113 reflektierter Anteil 112' der vom Emitter 110 emittierten elektromagnetischen Strahlung 112 von dem Empfänger 111 empfangbar ist. Dies ist in Figur 11 schematisch mittels der Pfeilrichtungen der emittierten elektromagnetischen Strahlung 112 und der an der Flüssigkeitsoberfläche 113 reflektierten elektromagnetischen Strahlung 112' angedeutet.

Wie ebenfalls in Figur 11 zu erkennen ist, kann zumindest einer von dem Emitter 110 und dem Empfänger 111 an einem Behälterboden 114 des Behälters 10 angeordnet sein. In dem hier abgebildeten Ausführungsbeispiel sind sowohl der Emitter 110 als auch der Empfänger 111 an dem Behälterboden 114 angeordnet, und zwar behälterinnenseitig, d.h. im Innenraum des Behälters 10, beziehungsweise an der dem Behälterinnenraum zugewandten Seite des Behälterbodens 114.

Es ist aber ebenso denkbar, dass zumindest einer von dem Emitter 110 und dem Empfänger 111 behälteraußenseitig an einem Behälterboden 114 des Behälters 10 angeordnet sei. In diesem Falle wären der Emitter 110 und/oder der Empfänger 111 auf der dem Innenraum des Behälters 10 abgewandten Seite des Behälterbodens 114 angeordnet.

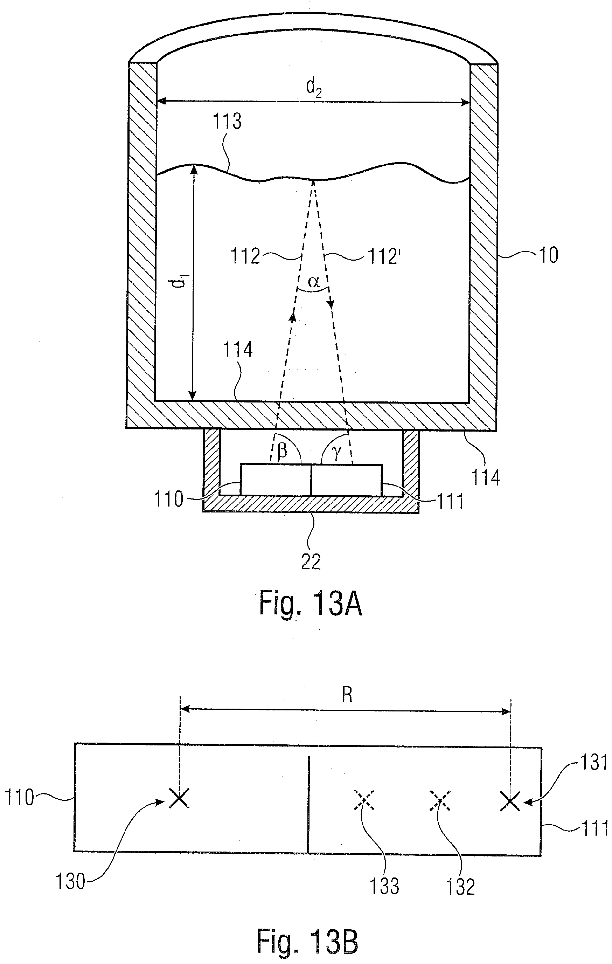

Ein solches Beispiel ist unter anderem in Figur 13A gezeigt. Beispielsweise kann der Behälter 10 einen bereits oben beschriebenen Vorsprung 22 aufweisen. Der Emitter 110 und/oder der Empfänger 111 können in diesem Vorsprung 22 angeordnet sein. Dabei kann der Vorsprung 22 als Teil des Behälterbodens 114 angesehen werden.

In diesem sowie allen anderen zuvor beschriebenen Ausführungsbeispielen kann der Behälterboden 114 aus einem für die elektromagnetische Strahlung 112 zumindest teilweise durchlässigen Material bestehen. Beispielsweise kann der Behälterboden 114 aus Glas (z.B. Plexiglas, Mineralglas) ausgeführt sein.

Das System 2" kann einen oder mehrere Empfänger 111 aufweisen. Beispielsweise kann ein solcher Empfänger 111 als eine Photodiode oder ein Lichtsensor ausgeführt sein. Der Empfänger 111 kann direkt an den Emitter 110 angrenzend, oder von dem Emitter 110 beabstandet angeordnet sein.

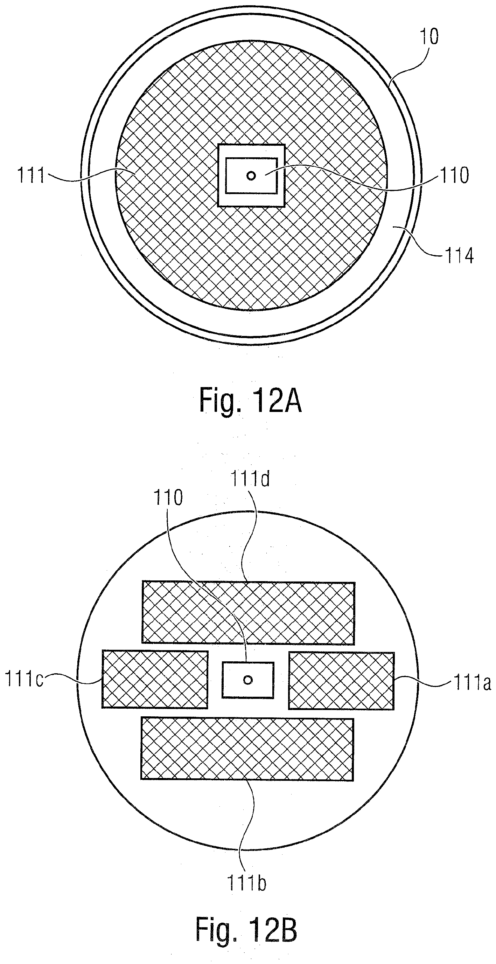

In Figur 12A ist ein Ausführungsbeispiel für die Anordnung von Emitter 110 und Empfänger gezeigt. Figur 12A zeigt eine Draufsicht auf einen Behälter 10. Behälterinnenseitig ist hier an dem Behälterboden 114 ein Emitter 110 angeordnet. Der Emitter 110 ist etwa mittig im Behälterboden 114 angeordnet.

Um den Emitter 110 herum ist ein Empfänger 111 angeordnet. Die viereckige Form des Emitters 110 sowie die runde Form des Empfängers 111 sind hier lediglich beispielhaft abgebildet. Sowohl der Emitter 110 als auch der Empfänger 111 können beliebige geometrische Formen aufweisen.

Der Empfänger 111 kann, wie in Figur 12A abgebildet, ein Flächensensor sein. Es wäre aber auch denkbar, dass der Empfänger 111 mehrere einzelne Punktsensoren aufweist. Ebenso denkbar wäre es, dass mehrere Empfänger 111a bis 111d um den Emitter 110 herum angeordnet sind, die wiederum beispielsweise in Form eines Arrays zusammengeschaltet sein können, wie dies zum Beispiel in Figur 12B abgebildet ist.

Wie eingangs erwähnt und beispielsweise in Figur 13A gezeigt ist, kann also der Emitter 110 derart an dem Behälter 10 angeordnet und ausgerichtet sein, dass die emittierte elektromagnetische Strahlung 112 auf die Flüssigkeitsoberfläche 113 der in dem Behälter 10 befindlichen Flüssigkeit auftrifft. Der Empfänger 111 wiederum kann derart an dem Behälter 10 angeordnet und ausgerichtet sein, dass der Empfänger 111 die an der Flüssigkeitsoberfläche 113 reflektierte elektromagnetische Strahlung 112' empfängt.

Der Emitter 110 kann die elektromagnetische Strahlung 112 in einem bestimmten Austrittswinkel $\beta$ schräg zur Flüssigkeitsoberfläche 113 emittieren. Die auf die Flüssigkeitsoberfläche 113 auftreffende elektromagnetische Strahlung 112 wird an der Grenze zwischen der Flüssigkeitsoberfläche 113 und der äußeren Umgebung (in der Regel Luft) reflektiert. Die reflektierte elektromagnetische Strahlung 112' trifft unter einem bestimmten Einfallwinkel $\gamma$ auf dem Empfänger 111 auf, wobei auch der Einfallwinkel $\gamma$ schräg zur Flüssigkeitsoberfläche 113, d.h. in einem von 90° unterschiedlichen Winkel, verläuft.

An der Flüssigkeitsoberfläche 113 stellt sich zwischen der emittierten elektromagnetischen Strahlung 112 und der reflektierten elektromagnetischen Strahlung 112' ein Reflexionswinkel $\alpha$ ein. Der Reflexionswinkel $\alpha$, der Austrittswinkel $\beta$ sowie der Einfallwinkel $\gamma$ sind voneinander abhängig gemäß der mathematischen Beziehung $\alpha + \beta + \gamma = 180°$, wobei $\beta$ und $\gamma$ im Idealfall (z.B. wenn der Behälter 10 gerade auf einer ebenen Fläche steht) als identisch angenommen werden können, da sich in diesem Idealfall ein gleichseitiges Dreieck zwischen der emittierten elektromagnetischen Strahlung 112 und der reflektierten elektromagnetischen Strahlung 112' bildet.

In dem in Figur 13A abgebildeten Beispiel ist die horizontale bzw. Azimuth-Lage der Flüssigkeitsoberfläche 113 relativ zu dem Füllstandssensor 101 in etwa parallel. Das heißt, wenn der Behälter wie in Figur 13A abgebildet, gerade steht, dann sind der Emitter 110 und der Empfänger 111 parallel zu der Flüssigkeitsoberfläche 113 ausgerichtet.

Sofern sich zwar in diesem Fall die Füllstandshöhe $d_1$, nicht aber die horizontale bzw. Azimuth-Lage der Flüssigkeitsoberfläche 113 relativ zu dem Füllstandssensor 101 ändert, bleiben auch die Winkel, d.h. Reflexionswinkel $\alpha$, Austrittswinkel $\beta$ und Einfallwinkel $\gamma$ vom Betrag her gleich. Es ändert sich jedoch mit variierendem Flüssigkeitspegel in dem Behälter 10 ein geometrischer Abstand zwischen einem Ort des Aussendens der emittierten elektromagnetischen Strahlung und einem Ort des Empfangs der reflektierten elektromagnetischen Strahlung. Dies soll beispielhaft mit Bezug auf Figur 13B erläutert werden.

Figur 13B zeigt eine vergrößerte schematische Draufsicht auf einen Füllstandsmesser 101 mit einem Emitter 110 und einem Empfänger 111. Das Kreuz 130 symbolisiert einen Austrittspunkt der emitierten elektromagnetischen Strahlung 112. Das Kreuz 131 symbolisiert einen Auftreffpunkt der reflektierten elektromagnetischen Strahlung 112' auf dem Empfänger 111. Der Austrittspunkt 130 und der Auftreffpunkt 131 sind um ein Maß R voneinander beabstandet.

Mit sinkender Füllstandshöhe $d_1$ im Behälter 10 verringert sich der Abstand R zwischen dem Austrittspunkt 130 und dem Auftreffpunkt 131. So läge beispielsweise bei einer im Vergleich zur ersten Füllstandshöhe niedrigeren zweiten Füllstandshöhe der Auftreffpunkt der reflektierten Strahlung 112' bei dem in Strichlinien dargestellten zweiten Kreuz 132. Und bei einer im Vergleich zur zweiten Füllstandshöhe noch niedrigeren dritten Füllstandshöhe läge der Auftreffpunkt der reflektierten Strahlung 112' beispielsweise bei dem in Strichlinien dargestellten dritten Kreuz 133.

Bei einem solchen Ausführungsbeispiels könnte der Füllstandsmesser 101 mit der bereits zuvor erwähnten Steuereinrichtung 102 (Figur 10) gekoppelt sein, wobei die Steuereinrichtung 102 ausgebildet ist, um den Füllstand der in dem Behälter 10 befindlichen Flüssigkeit unter Einbeziehung eines geometrischen Abstands R zwischen einem Ort 130 des Aussendens der emittierten elektromagnetischen Strahlung 112 und einem Ort 131 des Empfangs der reflektierten elektromagnetischen Strahlung 112' zu bestimmen.

Ein Vorteil der Erfindung liegt darin, dass auch bei einer Änderung der Azimuth-Lage der Flüssigkeitsoberfläche 113 relativ zu dem Emitter 110 und/oder Empfänger 111 die Füllstandshöhe mittels des erfindungsgemäßen Füllstandsmessers 101 bestimmbar ist.

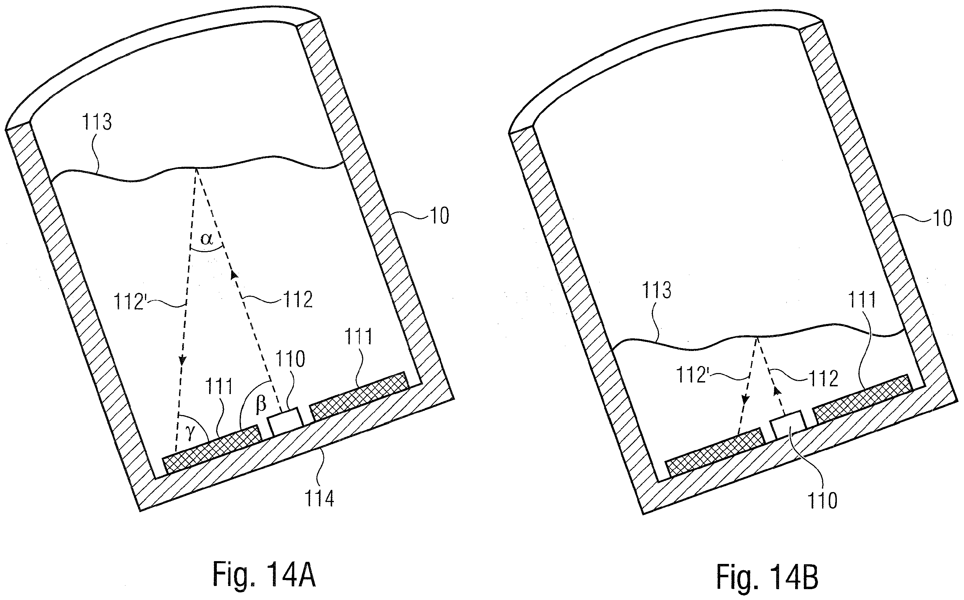

So zeigt beispielsweise Figur 14A ein weiteres Ausführungsbeispiel. Der Behälter 10 weist auf der dem Behälterinnenraum zugewandten Seite des Behälterbodens 114 einen Emitter 110 und einen um den Emitter 110 herum angeordneten Empfänger 111 auf.

Der Behälter 10 ist im Vergleich zu dem in Figur 13A abgebildeten Behälter 10 geneigt, was beispielsweise bei einer Trinkbewegung geschehen kann. Dadurch ändert sich die Azimuth-Lage der Flüssigkeitsoberfläche 113 relativ zu dem Emitter 110 und/oder Empfänger 111. Dadurch ist der Reflexionswinkel α im Vergleich zu dem in Figur 13A abgebildeten Beispiel größer, bei gleichbleibendem Austrittswinkel β des Emitters 110. Der Einfallwinkel γ ist hingegen kleiner als bei dem in Figur 13A abgebildeten Beispiel, bei gleichbleibendem Austrittswinkel β des Emitters 110. Außerdem liegt der Auftreffpunkt 131 der reflektierten elektromagnetischen Strahlung 112' auf dem Empfänger 111 weiter außen im Vergleich zu dem in Figur 13A abgebildeten ungeneigten Behälter 10, d.h. der geometrische Abstand R ist größer.

Zur Berücksichtigung dieser soeben beschriebenen Umstände bei einem geneigten Behälter 10, kann die Steuereinrichtung 102 optional zusätzlich mit einem Neigungssensor 46 gekoppelt sein. Der Neigungssensor 46 misst die Neigung des Behälters 10 und kalibriert die Füllstandsmessung der in dem Behälter 10 befindlichen Flüssigkeit um einen bestimmten Wert, der dem Betrag der Neigung des Behälters 10 entspricht.

Gemäß eines solchen Ausführungsbeispiels kann also die Steuereinrichtung 102 optional mit einem eine Neigung des Behälters 10 messenden Neigungssensor 46 gekoppelt sein, und die Steuereinrichtung 102 kann ferner ausgebildet sein, um den Füllstand der in dem Behälter 10 befindlichen Flüssigkeit unter Einbeziehung der gemessenen Neigung zu bestimmen.

Der Vollständigkeit halber zeigt Figur 14B den Behälter 10 in derselben Winkellage wie in Figur 14A. Allerdings ist der Füllstand der im Behälter 10 befindlichen Flüssigkeit im Vergleich zu Figur 14A gesunken. Wie eingangs erwähnt wurde, bleiben bei gleichbleibender Winkellage des Füllstandsmessers 101 (d.h. Emitter 110 und/oder Empfänger 111) relativ zur Flüssigkeitsoberfläche 113 zwar der Reflexionswinkel α, der Austrittswinkel β und der Einfallwinkel γ gleich. Es ändert sich jedoch der geometrische Abstand R zwischen dem Ort 130 des Aussendens der emittierten elektromagnetischen Strahlung 112 und dem Ort 131 des Empfangs der reflektierten elektromagnetischen Strahlung 112'. Dementsprechend ist in dem in Figur 14B gezeigten Beispiel der geometrische Abstand R geringer als bei dem in Figur 14A gezeigten Beispiel.

Mittels der oben genannten geometrischen Beziehungen, und der Möglichkeit zur Korrektur der Füllstandsmessung bei einer Neigung des Behälters 10 mittels des Neigungssensors 46, kann der Füllstandsmesser 101 den Füllstand der in dem Behälter 10 befindlichen Flüssigkeit sowohl bei gerade als auch bei geneigt ausgerichtetem Behälter 10 bestimmen. Dies bietet den Vorteil, dass der Füllstand des Behälters 10 beispielsweise auch während eines Trinkvorgangs messbar ist.

Beispielsweise kann über die Beziehung der Austritts-, Reflexions- und Einfallwinkel α, β, γ, sowie in Kenntnis des geometrischen Abstands R zwischen Austrittspunkt 130 und Auftreffpunkt 131, der Neigung des Behälters 10 sowie des Volumens $V_{Behälter}$ des Behälters 10 der Abstand $d_1$ zwischen der Flüssigkeitsoberfläche 113 und dem behälterinnenseitigen Behälterboden 114 bestimmt werden. Dieser Abstand $d_1$ entspricht der aktuellen Füllstandshöhe.

Beispielsweise kann bei einem gerade stehenden Behälter 10, in Kenntnis des Volumens $V_{Behälter}$ des Behälters 10, der Füllstand des Behälters 10 berechnet werden. Handelt es sich bei dem Behälter 10 beispielsweise um einen Kreiszylinder, wie in Figur 13A abgebildet, so kann die Füllstandshöhe $d_1$ z.B. gemäß folgender Formel berechnet werden:

$$V_{Behälter} = \pi \cdot \left(\frac{d_2}{2}\right)^2 \cdot d_1$$

Sofern zumindest einer der Winkel, d.h. Austrittswinkel β, Reflexionswinkel α und Einfallwinkel γ sowie der geometrische Abstand R bestimmt wurden bzw. bekannt sind, kann die Füllstandshöhe $d_1$ außerdem mittels der Winkelbeziehungen wie folgt ermittelt werden:

$$d_1 = \tan\gamma \cdot R$$

Alternativ oder zusätzlich kann der Füllstandsmesser 101 den Füllstand der in dem Behälter 10 befindlichen Flüssigkeit auch per Laufzeitmessung bestimmen. Hierfür kann der Füllstandsmesser 101 mit der bereits zuvor erwähnten Steuereinrichtung 102 gekoppelt sein, die ausgebildet ist, um den Füllstand der in dem Behälter 10 befindlichen Flüssigkeit unter Einbeziehung einer Laufzeitmessung der elektromagnetischen Strahlung 112 zwischen dem Emitter 110 und dem Empfänger 111 zu bestimmen.

Es wird hierbei die Laufzeit der vom Emitter 110 emittierten elektromagnetischen Strahlung 112 bis zum Auftreffen der reflektierten elektromagnetischen Strahlung 112' auf dem Empfänger gemessen. Je geringer der Flüssigkeitsstand im Behälter 10 ist, desto kürzer ist die gemessene Laufzeit, da die emittierte elektromagnetische Strahlung früher an der Flüssigkeitsoberfläche 13 reflektiert wird. Die Steuereinrichtung 102 ist hierbei ausgebildet, um mittels der gemessenen Laufzeit den Füllstand zu Berechnen.

Die oben beschriebenen erfindungsgemäßen Systeme 2, 2', 2'' können außerdem einen Flüssigkeitsdetektor 103 aufweisen. Mittels des Flüssigkeitsdetektors 103 kann festgestellt werden, ob sich Flüssigkeit in dem Behälter 10 befindet. Falls keine Flüssigkeit im Behälter 10 detektiert wird, kann beispielsweise darauf geschlossen werden, dass der Behälter 10 (z.B. nachts) derzeit nicht in Gebrauch ist. Elektronische Komponenten können dann in einen Standby-Modus geschaltet werden.

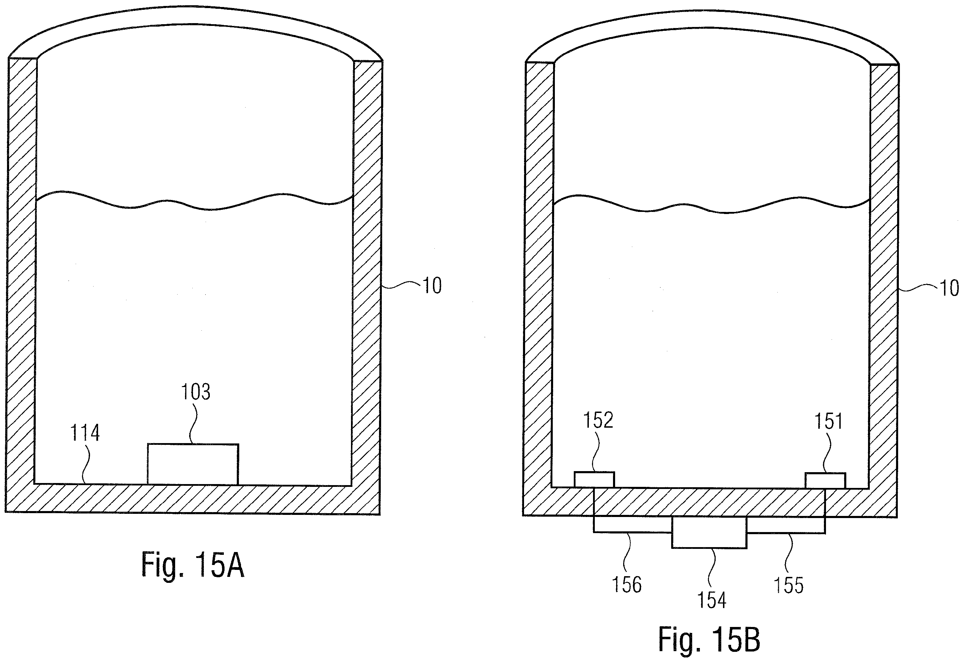

Figur 15A zeigt ein Ausführungsbeispiel eines solchen Systems mit einem Behälter 10, wobei der Behälter 10 einen Flüssigkeitsdetektor 103 aufweist, der ausgebildet ist, um zu detektieren, ob sich Flüssigkeit in dem Behälter 10 befindet. Wie in Figur 15A zu sehen ist, kann der Flüssigkeitsdetektor 103 zumindest abschnittsweise mit einer in dem Behälter 10 zu detektierenden Flüssigkeit in Kontakt sein.

Beispielsweise kann der Flüssigkeitsdetektor 103 an dem Behälterboden 114 angeordnet sein. Dabei kann der Flüssigkeitsdetektor 103 zum Beispiel, wie in Figur 15A abgebildet ist, auf der dem Behälterinnenraum zugewandten Seite des Behälterbodens 114 angeordnet sein. In diesem Fall ist der Flüssigkeitsdetektor 103 direkt mit der im Behälter 10 befindlichen Flüssigkeit in Kontakt.

Figur 15B zeigt ein weiteres Ausführungsbeispiel eines Flüssigkeitsdetektors 103. Hier weist der Flüssigkeitsdetektor 103 einen ersten elektrischen Kontakt 151 und einen zweiten elektrischen Kontakt 152 auf. Die beiden elektrischen Kontakte 151, 152 sind voneinander beabstandet angeordnet. Die elektrischen Kontakte 151, 152 sind derart ausgebildet, dass ein Stromkreis zwischen diesen beiden Kontakten 151, 152 mittels der in dem Behälter 10 zu detektierenden Flüssigkeit schließbar ist.

Beispielsweise ist der erste elektrische Kontakt 151 mittels einer elektrischen Leitung 156 mit einer Signalschaltung 154 verbunden. Der zweite elektrische Kontakt 152 ist mittels einer weiteren elektrischen Leitung 155 ebenfalls mit der Signalschaltung 154 verbunden. Die beiden elektrischen Kontakte 151, 152 sind zumindest abschnittsweise mit der in dem Behälter 10 zu detektierenden Flüssigkeit in Kontakt bringbar. In diesem Beispiel sind die beiden elektrischen Kontakte 151, 152 an der dem Behälterinnenraum zugewandten Seite des Behälterbodens 114 angeordnet.

Sofern sich keine Flüssigkeit in dem Behälter 10 befindet sind die elektrischen Kontakte 151, 152 nicht geschlossen. Es fließt kein Strom durch die Signalschaltung 154. Erst wenn sich Flüssigkeit in dem Behälter 10 befindet, dann wird der Stromkreis zwischen den beiden elektrischen Kontakten 151, 152 mittels dieser Flüssigkeit geschlossen. Somit fließt Strom durch die Signalschaltung 154 wodurch das Vorhandensein von Flüssigkeit in dem Behälter 10 detektiert werden kann.

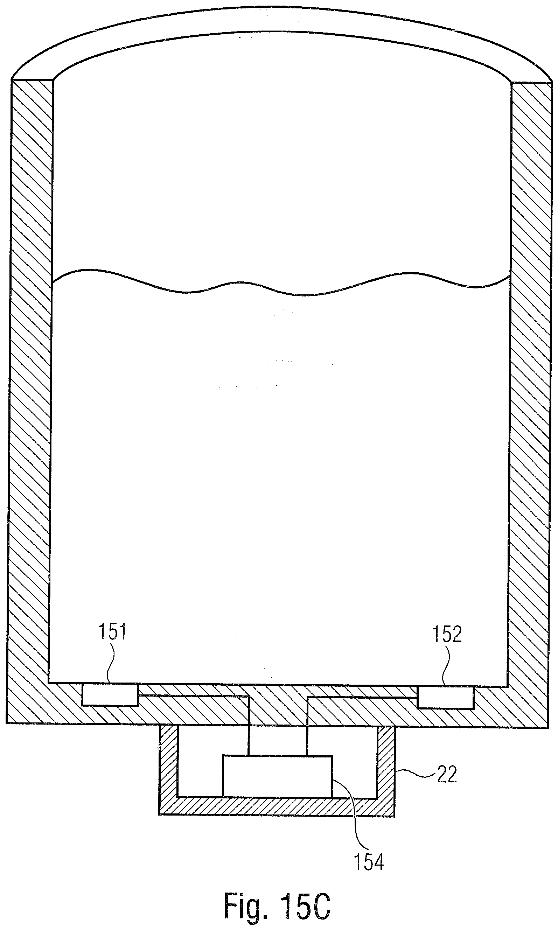

Gemäß eines Ausführungsbeispiels kann der Flüssigkeitsdetektor 103 in dem Behälterboden 114 integriert sein. Eine solche Anordnung ist in Figur 15C gezeigt. Hier weist der Behälter 10, als Teil des Behälterbodens 114, einen bereits zuvor ausführlich beschriebenen Vorsprung 22 auf. So kann beispielsweise die Signalschaltung 154 in dem Vorsprung 22 integriert sein.

Alternativ oder zusätzlich kann zumindest einer der beiden elektrischen Kontakte 151, 152 in dem Behälterboden 114, und insbesondere in der dem Behälterinnenraum zugewandten Seite des Behälterbodens 114, integriert sein. Die integrierten elektrischen Kontakte 151, 152 können beispielsweise bündig mit dem Behälterboden 114 fluchten.

Die beiden elektrischen Kontakte 151, 152 können, wie abgebildet, mit der Signalschaltung 154 gekoppelt sein. Die Signalschaltung 154 kann wiederum mit der Steuereinrichtung 102 gekoppelt sein. Die Signalschaltung 154 kann aber auch selbst ein Teil der Steuereinrichtung 102 sein.

Unter erneuter Bezugnahme auf Figur 10 kann also das erfindungsgemäße System 2'' eine Steuereinrichtung 102 aufweisen. Mit dieser Steuereinrichtung 102 wiederum können der oben beschriebene Füllstandsmesser 101, die Überwachungseinrichtung 4, die Animationseinrichtung 8, sowie optional ein Neigungssensor 46 und ein Flüssigkeitsdetektor 103 gekoppelt sein. Die Steuereinrichtung 102 kann die Steuerung des Füllstandsmessers 101 und/oder des Flüssigkeitsdetektors 103 sowie die Berechnungen der oben beschriebenen Füllstandsmessung und/oder Flüssigkeitsdetektion übernehmen.

Die Steuereinrichtung 102 selbst kann wiederum mit dem Behälter 10 oder mit dem oben beschriebenen Sockel 18 gekoppelt sein.

Die Kommunikation der Steuereinrichtung 102 mit dem Füllstandsmesser 101, der Überwachungseinrichtung 4, der Animationseinrichtung 8, dem Neigungssensor 46 und dem Flüssigkeitsdetektor 103 kann bidirektional sein. Die Steuereinrichtung 102 kann also Signale von den jeweiligen Elementen 101, 4, 8, 46, 103 empfangen und/oder Signale zu ebendiesen Elementen senden.

Die Animationseinrichtung 8 kann entweder direkt mit dem Benutzer 3 interagieren, wie mit der Transition 160 dargestellt, oder die Animationseinrichtung 8 kann mittels der Steuereinrichtung 102 mit dem Benutzer 3 interagieren, wie mit der Transition 161 dargestellt.

Anwendung kann das gezeigte System 2, 2', 2" z. B. im Altersheim finden, um älteren, beispielsweise bereits dementen Menschen eine Hilfestellung an die Hand zu geben, genügend Flüssigkeit am Tag aufzunehmen. Ferner können Firmen ihren Mitarbeitern das System zur Verfügung stellen, um Erkrankungen, die durch eine zu geringe Flüssigkeitsaufnahme ausgelöst werden können, vorzubeugen und so einen leistungsfähigeren Mitarbeiter haben. Ebenso kann das System in Zukunft zu einem Lifestyle-Artikel avancieren.

Nachfolgend werden weitere Ausführungsbeispiele genannt:

- Alle wesentlichen Mess-/Informations- und Signalfunktionen sollen bzw. können in der Basisstation /Sockel 18 untergebracht werden.
- Gefäß/Behälter 10, 10' kann integrierte Technik aufweisen.
- Erinnerungsfunktion durch Licht, Ton und Vibration (optisch, akustisch, haptisch etc.) an der Basis 18 mit Übertragung auf angeschlossenes Gefäß 10, 10' und Peripheriegeräte (Smart-Band, Smart-Watch etc.).
- Erinnerungsfunktion kann über das Gefäß/Behälter 10, 10' selbst erfolgen.
- Messfunktion für Füllstand/Gewicht (Erkennung des Gefäßes 10, 10' und dessen Nettogewichtes, Messung der entnommenen Flüssigkeitsmenge zur Überwachung der täglichen Trinkmenge/individuellen Mindestportion).
- Speicherung der Daten in der Basis/Sockel 18 und Synchronisierung mit Peripheriegeräten.
- Datenübertragung/Synchronisierung (Bluetooth, RFID, NFC bzw. andere kontaktlose Technik).
- Energieversorgung (autark, z.B. Solar, Energy Harvesting, Schwunggenerator etc.).

- Kopplung mit z. B. Smart-Band/-Watch u.ä. Gesundheits- und Vitalanalysegeräten (sowie alle Körpersensoren, die z.B. in Smart-Band/-Watch und ähnlichen Überwachungsgeräten genutzt werden, zur Überwachung der Vitalfunktionen, Informationsübermittlung Soll-/Ist-Menge, Programmierung der Funktionen und individuellen Parameter über App bzw. webbasiert über verschiedene Devices wie PC, Tablet, Smartphone etc.).
- Messung der Dehydrierung durch Sensoren (ggf. zur Früherkennung).
- Temperaturüberwachung (Schutz der Elektronik und Warnfunktion bei Heißgetränken etc.).
- Ladung der Speicherbatterie durch RFID/NFC.
- USB-Anschluss als Ladefunktion z.B. bei Tiefentladung des Energiespeichers und Programmierschnittstelle etc.
- Nutzung des USB-Anschlusses für Kühl-/Warmhaltefunktion (über Peltierelement, Infrarot, Induktion oder ähnliches).
- Basis 18 mit Kopplung an aufeinander abgestimmte, systemkonforme Gefäße 10, 10' (Becher, Flasche oder Gefäße, wie Karaffen etc. mit größerem Bodendurchmesser mit zusätzlichem Adapterring zwecks Standfestigkeit etc.) durch z. B. form- oder kraftschlüssige Verbindung (Bajonett, Magnet, Gewinde, Saugnapf o.ä.).
- Deaktivierung des Systems durch Abstellfunktion ("auf den Kopf stellen" etc.) um Energie zu sparen z.B. nachdem ein Warnsignal z.B. durch rotes Leuchten ausgegeben wurde um eine Beschädigung beispielsweise in einer Spülmaschine zu vermeiden.
- Deaktivierung des Systems bei Trennung des Sockels 18 vom Gefäß 10, 10', bzw. Aktivierung bei Verbindung mit dem Gefäß 10, 10' (stand-by).
- Kopplung und Erkennung verschiedener ggf. personalisierter/codierter Gefäße 10, 10'.
- Personalisierung der Sockel 18 und Gefäße 10, 10' (Paarung), Gefäß 10, 10' ggf. mit Chip, RFID, Strich-/QR-code etc.
- Erfassung und Analyse des Gefäßinhalts (Nährstoff-/Mineralgehalt, Kalorien etc.)
- Auslesestation zum Auslesen der Daten über handgeführte oder stationäre Technik (ggf. Kombination mit Ziffer einer Ladung der Speicherbatterie durch RFID/NFC) mit Personenzuordnung innerhalb Datenbanken.

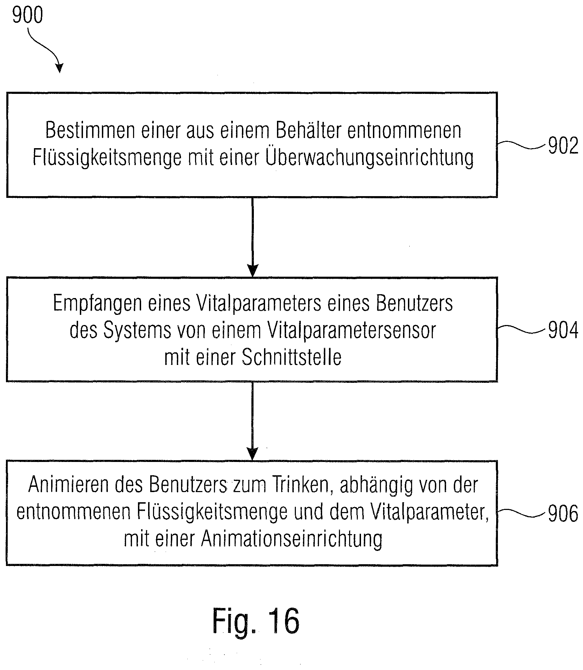

Fig. 16 zeigt eine schematische Darstellung eines Verfahrens 900 zum Betrieb eines Systems zum Überwachen einer Flüssigkeitsaufnahme eines Benutzers. Das Verfahren 100 weist einen Schritt 902 mit Bestimmen einer aus einem Behälter 10, 10' entnommenen Flüssigkeitsmenge mit einer Überwachungseinrichtung 4, einen Schritt 904 mit Empfangen eines Vitalparameters eines Benutzersystems von einem Vitalparametersensor mit einer Schnittstelle sowie einen Schritt 906 mit Animieren des Benutzers zum Trinken, abhängig von der entnommenen Flüssigkeitsmenge und dem Vitalparameter, mit einer Animationseinrichtung 8 auf.

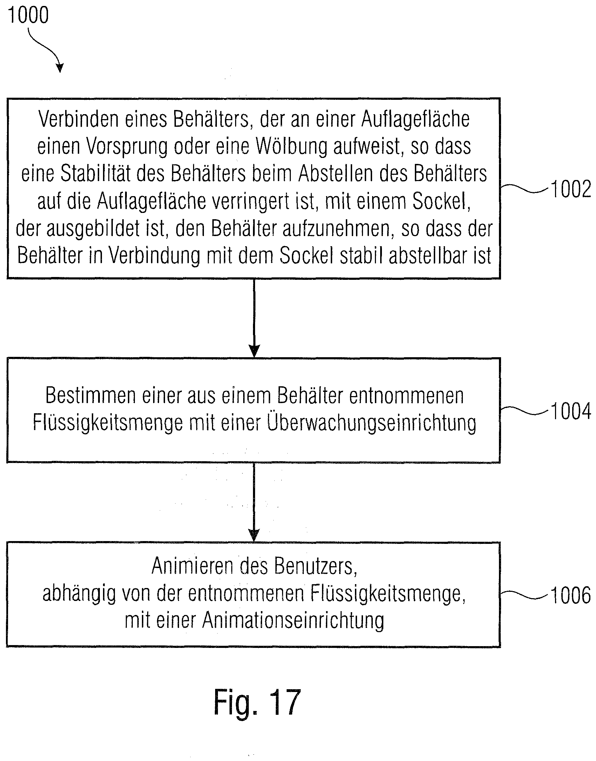

Fig. 17 zeigt ein schematisches Blockdiagramm eines Verfahrens 1000 zum Betrieb eines Systems zum Überwachen einer Flüssigkeitsaufnahme eines Benutzers. Das System 1000 umfasst einen Schritt 1002 mit Verbinden eines Behälters 10, 10', der an einer Auflagefläche einen Vorsprung 22 oder eine Wölbung aufweist, so dass eine Stabilität des Behälters 10, 10' beim Abstellen des Behälters 10, 10' auf die Auflagefläche verringert ist, mit einem Sockel 18, der ausgebildet ist, um den Behälter 10, 10' aufzunehmen, so dass der Behälter 10, 10' in Verbindung mit dem Sockel 18 stabil abstellbar ist, einen Schritt 1004 mit Bestimmen einer aus dem Behälter 10, 10' entnommenen Flüssigkeitsmenge mit einer Überwachungseinrichtung 4 sowie einen Schritt 1006 mit Animieren des Benutzers, abhängig von der entnommenen Flüssigkeitsmenge, mit einer Animationseinrichtung 8.

Obwohl manche Aspekte im Zusammenhang mit einer Vorrichtung beschrieben wurden, versteht es sich, dass diese Aspekte auch eine Beschreibung des entsprechenden Verfahrens darstellen, sodass ein Block oder ein Bauelement einer Vorrichtung auch als ein entsprechender Verfahrensschritt oder als ein Merkmal eines Verfahrensschrittes zu verstehen ist. Analog dazu stellen Aspekte, die im Zusammenhang mit einem oder als ein Verfahrensschritt beschrieben wurden, auch eine Beschreibung eines entsprechenden Blocks oder Details oder Merkmals einer entsprechenden Vorrichtung dar. Einige oder alle der Verfahrensschritte können durch einen Hardware-Apparat (oder unter Verwendung eines Hardware-Apparats), wie zum Beispiel einen Mikroprozessor, einen programmierbaren Computer oder einer elektronischen Schaltung durchgeführt werden. Bei einigen Ausführungsbeispielen können einige oder mehrere der wichtigsten Verfahrensschritte durch einen solchen Apparat ausgeführt werden.

Je nach bestimmten Implementierungsanforderungen können Ausführungsbeispiele der Erfindung in Hardware oder in Software implementiert sein. Die Implementierung kann unter Verwendung eines digitalen Speichermediums, beispielsweise einer Floppy-Disk, einer DVD, einer BluRay Disc, einer CD, eines ROM, eines PROM, eines EPROM, eines EEPROM oder eines FLASH-Speichers, einer Festplatte oder eines anderen magnetischen oder optischen Speichers durchgeführt werden, auf dem elektronisch lesbare Steuersignale gespeichert sind, die mit einem programmierbaren Computersystem derart zusammenwirken können oder zusammenwirken, dass das jeweilige Verfahren durchgeführt wird. Deshalb kann das digitale Speichermedium computerlesbar sein.

Manche Ausführungsbeispiele gemäß der Erfindung umfassen also einen Datenträger, der elektronisch lesbare Steuersignale aufweist, die in der Lage sind, mit einem programmierbaren Computersystem derart zusammenzuwirken, dass eines der hierin beschriebenen Verfahren durchgeführt wird.

Allgemein können Ausführungsbeispiele der vorliegenden Erfindung als Computerprogrammprodukt mit einem Programmcode implementiert sein, wobei der Programmcode dahin gehend wirksam ist, eines der Verfahren durchzuführen, wenn das Computerprogrammprodukt auf einem Computer abläuft.

Der Programmcode kann beispielsweise auch auf einem maschinenlesbaren Träger gespeichert sein.

Andere Ausführungsbeispiele umfassen das Computerprogramm zum Durchführen eines der hierin beschriebenen Verfahren, wobei das Computerprogramm auf einem maschinenlesbaren Träger gespeichert ist. Mit anderen Worten ist ein Ausführungsbeispiel des erfindungsgemäßen Verfahrens somit ein Computerprogramm, das einen Programmcode zum Durchführen eines der hierin beschriebenen Verfahren aufweist, wenn das Computerprogramm auf einem Computer abläuft.

Ein weiteres Ausführungsbeispiel der erfindungsgemäßen Verfahren ist somit ein Datenträger (oder ein digitales Speichermedium oder ein computerlesbares Medium), auf dem das Computerprogramm zum Durchführen eines der hierin beschriebenen Verfahren aufgezeichnet ist.

Ein weiteres Ausführungsbeispiel des erfindungsgemäßen Verfahrens ist somit ein Datenstrom oder eine Sequenz von Signalen, der bzw. die das Computerprogramm zum Durchführen eines der hierin beschriebenen Verfahren darstellt bzw. darstellen. Der Datenstrom oder die Sequenz von Signalen kann bzw. können beispielsweise dahin gehend konfiguriert sein, über eine Datenkommunikationsverbindung, beispielsweise über das Internet, transferiert zu werden.

Ein weiteres Ausführungsbeispiel umfasst eine Verarbeitungseinrichtung, beispielsweise einen Computer oder ein programmierbares Logikbauelement, die dahin gehend konfiguriert oder angepasst ist, eines der hierin beschriebenen Verfahren durchzuführen.

Ein weiteres Ausführungsbeispiel umfasst einen Computer, auf dem das Computerprogramm zum Durchführen eines der hierin beschriebenen Verfahren installiert ist.

Ein weiteres Ausführungsbeispiel gemäß der Erfindung umfasst eine Vorrichtung oder ein System, die bzw. das ausgelegt ist, um ein Computerprogramm zur Durchführung zumindest eines der hierin beschriebenen Verfahren zu einem Empfänger zu übertragen. Die Übertragung kann beispielsweise elektronisch oder optisch erfolgen. Der Empfänger kann beispielsweise ein Computer, ein Mobilgerät, ein Speichergerät oder eine ähnliche Vorrichtung sein. Die Vorrichtung oder das System kann beispielsweise einen Datei-Server zur Übertragung des Computerprogramms zu dem Empfänger umfassen.

Bei manchen Ausführungsbeispielen kann ein programmierbares Logikbauelement (beispielsweise ein feldprogrammierbares Gatterarray, ein FPGA) dazu verwendet werden, manche oder alle Funktionalitäten der hierin beschriebenen Verfahren durchzuführen. Bei manchen Ausführungsbeispielen kann ein feldprogrammierbares Gatterarray mit einem Mikroprozessor zusammenwirken, um eines der hierin beschriebenen Verfahren durchzuführen. Allgemein werden die Verfahren bei einigen Ausführungsbeispielen seitens einer beliebigen Hardwarevorrichtung durchgeführt. Diese kann eine universell einsetzbare Hardware wie ein Computerprozessor (CPU) sein oder für das Verfahren spezifische Hardware, wie beispielsweise ein ASIC.

Die oben beschriebenen Ausführungsbeispiele stellen lediglich eine Veranschaulichung der Prinzipien der vorliegenden Erfindung dar. Es versteht sich, dass Modifikationen und Variationen der hierin beschriebenen Anordnungen und Einzelheiten anderen Fachleuten einleuchten werden. Deshalb ist beabsichtigt, dass die Erfindung lediglich durch den Schutzumfang der nachstehenden Patentansprüche und nicht durch die spezifischen Einzelheiten, die anhand der Beschreibung und der Erläuterung der Ausführungsbeispiele hierin präsentiert wurden, beschränkt sei.

**Patentansprüche**

1. System (2") zum Überwachen einer Flüssigkeitsaufnahme eines Benutzers (3) mit folgenden Merkmalen:

   einem Behälter (10, 10') zum Aufnehmen von Flüssigkeit;
   einen Füllstandsmesser (101) zum Messen des Füllstands der in dem Behälter (10, 10') befindlichen Flüssigkeit, wobei der Füllstandsmesser (101) ein optischer Füllstandsmesser ist, der mindestens einen Emitter (110) zum Emittieren von elektromagnetischer Strahlung (112) und mindestens einen Empfänger (111) zum Empfangen der emittierten elektromagnetischen Strahlung (112) aufweist;
   eine Überwachungseinrichtung (4), die ausgebildet ist, um eine aus dem Behälter (10, 10') entnommene Flüssigkeitsmenge zu bestimmen; und
   eine Animationseinrichtung (8), die ausgebildet ist, um abhängig von der entnommenen Flüssigkeitsmenge den Benutzer (3) zum Trinken zu animieren,
   wobei der Emitter (110) derart an dem Behälter (10, 10') angeordnet ist, dass die emittierte elektromagnetische Strahlung (112) auf die zur Umgebung angrenzende Flüssigkeitsoberfläche (113) der in dem Behälter (10, 10') befindlichen Flüssigkeit auftrifft,
   wobei der Empfänger (111) derart an dem Behälter (10, 10') angeordnet ist, dass ein an der Flüssigkeitsoberfläche (113) reflektierter Anteil

(112') der vom Emitter (110) emittierten elektromagnetischen Strahlung von dem Empfänger (111) empfangbar ist; und
wobei zumindest einer von dem Emitter (110) und dem Empfänger (111) an einem Behälterboden (114) des Behälters (10, 10') angeordnet ist.

2. System (2") nach Anspruch 1, wobei der Emitter (110) mindestens einen Laser aufweist.

3. System (2") nach einem der Ansprüche 1 oder 2, wobei der Füllstandsmesser (101) mit einer Steuereinrichtung (102) gekoppelt ist, die ausgebildet ist, um den Füllstand der in dem Behälter (10, 10') befindlichen Flüssigkeit unter Einbeziehung einer Laufzeitmessung der elektromagnetischen Strahlung (112, 112') zwischen dem Emitter (110) und dem Empfänger (111) zu bestimmen.

4. System (2") nach einem der Ansprüche 1 bis 3, wobei der Füllstandsmesser (101) mit einer Steuereinrichtung (102) gekoppelt ist, die ausgebildet ist, um den Füllstand der in dem Behälter (10, 10') befindlichen Flüssigkeit unter Einbeziehung eines geometrischen Abstands R zwischen einem Ort (130) des Aussendens der emittierten elektromagnetischen Strahlung (112) und einem Ort (131) des Empfangs der elektromagnetischen Strahlung (112') zu bestimmen.

5. System (2") nach einem der Ansprüche 3 oder 4, wobei die Steuereinrichtung (102) mit einem eine Neigung des Behälters (10, 10') messenden Neigungssensor (46) gekoppelt ist, und wobei die Steuereinrichtung (102) ferner ausgebildet ist, um den Füllstand der in dem Behälter (10, 10') befindlichen Flüssigkeit unter Einbeziehung der gemessenen Neigung zu bestimmen.

6. System (2', 2") nach einem der vorhergehenden Ansprüche, mit einer Schnittstelle (6), die ausgebildet ist, um von einem Vitalparametersensor (12) einen Vitalparameter (14) des Benutzers zu empfangen, wobei die Animationseinrichtung (8) ausgebildet ist, um abhängig von der entnommenen Flüssigkeitsmenge und dem Vitalparameter (14), den Benutzer (3) zum Trinken zu animieren.

7. System (2, 2', 2") gemäß einem der vorherigen Ansprüche, wobei die Überwachungseinrichtung ausgebildet ist, um die aus dem Behälter (10, 10') entnommene Flüssigkeitsmenge (16) mittels einer kapazitiven Messung, mittels einer optischen Messung, mittels Ultraschall, mittels Radar, mittels einer Gewichtsänderung, mittels einer Laufzeitmessung und/oder mittels einer Leitfähigkeitsmessung zu ermitteln.

**8.** System (2, 2', 2") gemäß einem der vorherigen Ansprüche, wobei die Animationseinrichtung ausgebildet ist, um den Behälter (10, 10'), den Vitalparametersensor (12) oder ein mobiles Gerät (24) zu animieren, den Benutzer (3) visuell, auditiv oder taktil zum Trinken zu animieren.

**9.** System (2, 2', 2") gemäß einem der vorhergehenden Ansprüche, wobei der Behälter (10, 10') aus einem lichtdurchlässigen Material gebildet ist, wobei die Animationseinrichtung (8) ausgebildet ist, um zum Animieren Licht in den Behälter (10, 10') einzukoppeln, und wobei der Behälter (10, 10') Streuzentren (52) aufweist, die ausgebildet sind, um das eingekoppelte Licht zu streuen; oder
wobei die Animationseinrichtung (8) ausgebildet ist, um zum Animieren eine elektrische Spannung oder einen elektrischen Strom an den Behälter (10, 10') anzulegen und in dem Behälter (10, 10') eingebrachte oder auf dem Behälter (10, 10') aufgebrachte Animationsmittel anzuregen.

**10.** System (2, 2', 2") gemäß Anspruch 9, wobei der Behälter (10, 10') einen Vorsprung aufweist und wobei die Auswerteeinheit (8) ausgebildet ist, um das Licht an dem Vorsprung (22) in den Behälter (10, 10') einzukoppeln.

**11.** System (2, 2', 2") gemäß einem der vorherigen Ansprüche, wobei die Animationseinrichtung (8) in einem mobilen Gerät (24) angeordnet ist.

**12.** System (2, 2', 2") gemäß einem der vorherigen Ansprüche mit einer Trinkhilfe (35), wobei die Trinkhilfe (35) ausgebildet ist, um einen Durchfluss einer dem Behälter (10, 10') mittels der Trinkhilfe (35) entnommenen Flüssigkeitsmenge zu bestimmen und die dem Behälter (10, 10') mittels der Trinkhilfe (35) entnommenen Flüssigkeitsmenge der Überwachungseinrichtung (4) bereitzustellen.

**13.** System (2, 2', 2") gemäß einem der vorhergehenden Ansprüche, wobei der Behälter (10, 10') einen Flüssigkeitsdetektor aufweist, der ausgebildet ist, um zu detektieren, ob sich Flüssigkeit in dem Behälter (10, 10') befindet, wobei der Flüssigkeitsdetektor zumindest abschnittsweise mit einer in dem Behälter (10, 10') zu detektierenden Flüssigkeit in Kontakt bringbar ist.

**14.** System (2, 2', 2") nach Anspruch 13, wobei der Flüssigkeitsdetektor mindestens zwei voneinander beabstandete elektrische Kontakte aufweist, die derart ausgebildet sind, dass ein Stromkreis zwischen diesen beiden Kontakten mittels der in dem Behälter (10, 10') zu detektierenden Flüssigkeit schließbar ist.

**15.** System (2, 2', 2") nach einem der Ansprüche 13 oder 14, wobei der Flüssigkeitsdetektor in einem Behälterboden des Behälters (10, 10') integriert ist.

**Claims**

**1.** System (2") for monitoring liquid intake of a user (3), comprising:

a container (10, 10') for receiving liquid;
a level meter (101) for measuring the filling level of the liquid contained within the container (10, 10'), wherein the level meter (101) is an optical level meter comprising at least one emitter (110) for emitting electromagnetic radiation (112) and at least one receiver (111) for receiving the emitted electromagnetic radiation (112);
a monitoring means (4) configured to determine an amount of liquid removed from a container (10, 10'); and
a prompting means (8) configured to prompt the user (3) to drink as a function of the amount of liquid removed,
wherein the emitter (110) is arranged at the container (10, 10') such that the emitted electromagnetic radiation (112) impinges upon the liquid surface (113), adjoining the surroundings, of the liquid contained within the container (10, 10'),
wherein the receiver (111) is arranged at the container (10, 10') such that a portion (112'), reflected at the liquid surface (113), of the electromagnetic radiation emitted by the emitter (110) is receivable by the receiver (111); and
wherein at least one of the emitter (110) and the receiver (111) is arranged at a container bottom (114) of the container (10, 10').

**2.** System (2") as claimed in claim 1, wherein the emitter (110) comprises at least one laser.

**3.** System (2") as claimed in any of claims 1 or 2, wherein the level meter (101) is coupled to a control means (102) configured to determine the filling level of the liquid contained within the container (10, 10') while taking into account a run-time measurement of the electromagnetic radiation (112, 112') between the emitter (110) and the receiver (111).

**4.** System (2") as claimed in any of claims 1 to 3, wherein the level meter (101) is coupled to a control means (102) configured to determine the filling level of the liquid contained within the container (10, 10') while taking into account a geometric distance R between a location (130) of sending out the emitted electromagnetic radiation (112) and a location (131) of receiving the electromagnetic radiation (112').

**5.** System (2") as claimed in any of claims 3 or 4, wherein the control means (102) is coupled to an inclination sensor (46) measuring an inclination of the container (10, 10'), and wherein the control means (102) is further configured to determine the filling level of the liquid contained within the container (10, 10') while taking into account the inclination measured.

6. System (2', 2") as claimed in any of the previous claims, comprising an interface (6) configured to receive a vital parameter (14) of the user from a vital parameter sensor (12), wherein the prompting means (8) is configured to prompt the user (3) to drink as a function of the amount of liquid removed and of the vital parameter (14).

7. System (2, 2', 2") as claimed in any of the previous claims, wherein the monitoring means is configured to determine the amount of liquid removed (16) from the container (10, 10') by means of capacitive measurement, by means of optical measurement, by means of ultrasound, by means of radar, by means of a change in weight, by means of a run-time measurement, and/or by means of a conductivity measurement.

8. System (2, 2', 2") as claimed in any of the previous claims, wherein the prompting means is configured to prompt the container (10, 10'), the vital parameter sensor (12) or a mobile device (24) to prompt the user (3) to drink in a visual, auditive or tactile manner.

9. System (2, 2', 2") as claimed in any of the previous claims, wherein the container (10, 10') is formed of a transparent material, the prompting means (8) is configured to couple light into the container (10, 10') for prompting purposes, and the container (10, 10') comprising scattering centers (52) configured to scatter the light coupled in; or
wherein the prompting means (8) is configured to apply, for prompting purposes, an electric voltage or an electric current to the container (10, 10') and to excite the prompting means introduced into the container (10, 10') or applied onto the container (10, 10').

10. System (2, 2', 2") as claimed in claim 9, wherein the container (10, 10') comprises a projection, and wherein the evaluation unit (8) is configured to couple the light at the projection (22) into the container (10, 10').

11. System (2, 2', 2") as claimed in any of the previous claims, wherein the prompting means (8) is arranged inside a mobile device (24).

12. System (2, 2', 2") as claimed in any of the previous claims, comprising a drinking aid (35), the drinking aid (35) being configured to determine an amount of liquid flowing through which has been removed from the container (10, 10') by means of the drinking aid (35), and to provide the amount of liquid removed from the container (10, 10') by means of the drinking aid (35) to the monitoring means (4).

13. System (2, 2', 2") as claimed in any of the previous claims, wherein the container (10, 10') comprises a liquid detector configured to detect whether or not there is liquid contained within the container (10, 10'), which liquid detector may be brought into contact, at least in some portions, with a liquid to be detected within the container (10, 10').

14. System (2, 2', 2") as claimed in claim 13, wherein the liquid detector comprises at least two electric contacts spaced apart from each other and configured such that an electric circuit between said two contacts may be closed by means of the liquid to be detected within the container (10, 10').

15. System (2, 2', 2") as claimed in any of claims 13 or 14, wherein the liquid detector is integrated into a container bottom of the container (10, 10').

**Revendications**

1. Système (2') de surveillance d'une absorption de liquide d'un utilisateur (3), aux caractéristiques suivantes:

    un récipient (10, 10') destiné à recevoir du liquide;
    un indicateur de niveau de remplissage (101) destiné à mesurer le niveau de remplissage du liquide présent dans le récipient (10, 10'), où l'indicateur de niveau de remplissage (101) est un indicateur de niveau de remplissage optique qui présente au moins un émetteur (110) destiné à émettre un rayonnement électromagnétique (112) et au moins un récepteur (111) destiné à recevoir le rayonnement électromagnétique émis (112);
    un moyen de surveillance (4) qui est conçu pour déterminer une quantité de liquide prélevée du récipient (10, 10'); et
    un moyen d'animation (8) qui est conçu pour animer l'utilisateur (3) à boire en fonction de la quantité de liquide prélevée,
    dans lequel l'émetteur (110) est disposé sur le récipient (10, 10') de sorte que le rayonnement électromagnétique émis (112) tombe sur la surface de liquide adjacente à l'environnement (113) du liquide présent dans le récipient (10, 10'),
    dans lequel le récepteur (111) est disposé sur le récipient (10, 10') de sorte qu'une part (112') du rayonnement électromagnétique émis par l'émetteur (110) réfléchie sur la surface de liquide (113) puisse être reçue par le récepteur (111); et

dans lequel au moins un parmi l'émetteur (110) et le récepteur (111) est disposé sur un fond de récipient (114) du récipient (10, 10').

2. Système (2") selon la revendication 1, dans lequel l'émetteur (110) présente au moins un laser.

3. Système (2") selon l'une des revendications 1 ou 2, dans lequel l'indicateur de niveau (101) est couplé à un moyen de commande (102) qui est conçu pour déterminer le niveau de remplissage du liquide présent dans le récipient (10, 10') en tenant compte d'une mesure du temps de parcours du rayonnement électromagnétique (112, 112') entre l'émetteur (110) et le récepteur (111).

4. Système (2") selon l'une des revendications 1 à 3, dans lequel l'indicateur de niveau de remplissage (101) est couplé à un moyen de commande (102) qui est conçu pour déterminer le niveau de remplissage du liquide dans le récipient (10, 10') en tenant compte d'une distance géométrique R entre un endroit (130) de l'émission du rayonnement électromagnétique (112) et un endroit (131) de réception du rayonnement électromagnétique (112').

5. Système (2") selon la revendication 3 ou 4, dans lequel le moyen de commande (102) est couplé à un capteur d'inclinaison (46) mesurant une inclinaison du récipient (10, 10'), et dans lequel le moyen de commande (102) est par ailleurs conçu pour déterminer le niveau de remplissage du liquide dans le récipient (10, 10') en tenant compte de l'inclinaison mesurée.

6. Système (2', 2") selon l'une des revendications précédentes, avec une interface (6) qui est conçue pour recevoir d'un capteur de paramètres vitaux (12) un paramètre vital (14) de l'utilisateur, dans lequel le moyen d'animation (8) est conçu pour animer l'utilisateur (3) à boire en fonction de la quantité de fluide prélevée et du paramètre vital (14).

7. Système (2, 2', 2") selon l'une des revendications précédentes, dans lequel le moyen de surveillance est conçu pour déterminer la quantité de liquide (16) prélevée du récipient (10, 10') au moyen d'une mesure capacitive, au moyen d'une mesure optique, au moyen d'ultrasons, au moyen d'un radar, au moyen d'une variation de poids, au moyen d'une mesure du temps de parcours et/ou au moyen d'une mesure de conductivité.

8. Système (2, 2', 2") selon l'une des revendications précédentes, dans lequel le moyen d'animation est conçu pour animer le récipient (10, 10'), le capteur de paramètres vitaux (12) ou un appareil mobile (24), pour animer de manière visuelle, auditive ou tactile l'utilisateur (3) à boire.

9. Système (2, 2', 2") selon l'une des revendications précédentes, dans lequel le récipient (10, 10') est formé à partir d'un matériau translucide, dans lequel le moyen d'animation (8) est conçu pour introduire de la lumière dans le récipient (10, 10') pour l'animation, et dans lequel le récipient (10, 10') présente des centres de diffusion (52) qui sont conçus pour diffuser la lumière introduite; ou
dans lequel le moyen d'animation (8) est conçu pour appliquer une tension électrique ou un courant électrique au récipient (10, 10') pour l'animation et pour exciter les moyens d'animation introduits dans le récipient (10, 10') ou placés sur le récipient (10, 10').

10. Système (2, 2', 2") selon la revendication 9, dans lequel le récipient (10, 10') présente une saillie et dans lequel l'unité d'évaluation (8) est conçue pour introduire la lumière à la saillie (22) dans le récipient (10, 10').

11. Système (2, 2', 2") selon l'une des revendications précédentes, dans lequel le moyen d'animation (8) est disposé dans un appareil mobile (24).

12. Système (2, 2', 2") selon l'une des revendications précédentes avec un moyen d'aide pour boire (35), dans lequel le moyen d'aide pour boire (35) est conçu pour déterminer un passage d'une quantité de liquide prélevée du récipient (10, 10') au moyen du moyen d'aide pour boire (35) et pour fournir au moyen de surveillance (4) la quantité de liquide prélevée du récipient (10, 10') au moyen du moyen d'aide pour boire (35).

13. Système (2, 2', 2") selon l'une des revendications précédentes, dans lequel le récipient (10, 10') présente un détecteur de liquide qui est conçu pour détecter s'il y a du liquide dans le récipient (10, 10'), dans lequel le détecteur de liquide peut être amené au moins par segments en contact avec un liquide à détecter dans le récipient (10, 10').

14. Système (2, 2', 2") selon la revendication 13, dans lequel le détecteur de liquide présente au moins deux contacts électriques distants l'un de l'autre qui sont conçus de sorte qu'un circuit de courant entre ces deux contacts puisse être fermé au moyen du liquide à détecter dans le récipient (10, 10').

15. Système (2, 2', 2") selon l'une des revendications 13 ou 14, dans lequel le détecteur de liquide est intégré dans un fond du récipient (10, 10').

EP 3 711 660 B1

**10**

**2**

**16**

**4** Überwachungseinrichtung

**16**

**14** Vitalparametersensor

**12**

**14** Schnittstelle

**6**

**16**

Animationseinrichtung

**8**

**17** Benutzer

**3**

Fig. 1

Fig. 2

EP 3 711 660 B1

Fig. 3A

Fig. 3B

Fig. 3C

Fig. 4

Fig. 5

Fig. 6A

Fig. 6B

Fig. 6C

Fig. 6D

Fig. 6E

10, 10'

18

48

30

## Fig. 7

Fig. 8

Fig. 9

Fig. 10

**Fig. 11**

Fig. 12A

Fig. 12B

Fig. 13A

Fig. 13B

Fig. 14B

Fig. 14A

Fig. 15A

Fig. 15B

Fig. 15C

900

Bestimmen einer aus einem Behälter entnommenen
Flüssigkeitsmenge mit einer Überwachungseinrichtung — 902

Empfangen eines Vitalparameters eines Benutzers
des Systems von einem Vitalparametersensor
mit einer Schnittstelle — 904

Animieren des Benutzers zum Trinken, abhängig von der
entnommenen Flüssigkeitsmenge und dem Vitalparameter,
mit einer Animationseinrichtung — 906

Fig. 16

1000

Verbinden eines Behälters, der an einer Auflagefläche
einen Vorsprung oder eine Wölbung aufweist, so dass
eine Stabilität des Behälters beim Abstellen des Behälters
auf die Auflagefläche verringert ist, mit einem Sockel,
der ausgebildet ist, den Behälter aufzunehmen, so dass der
Behälter in Verbindung mit dem Sockel stabil abstellbar ist

— 1002

Bestimmen einer aus einem Behälter entnommenen
Flüssigkeitsmenge mit einer Überwachungseinrichtung

— 1004

Animieren des Benutzers,
abhängig von der entnommenen Flüssigkeitsmenge,
mit einer Animationseinrichtung

— 1006

Fig. 17

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2014046596 A1
- US 2015359364 A1